# EUROPEAN PATENT SPECIFICATION

(11) **EP 4 578 488 B1**
(45) Date of publication and mention of the grant of the patent: **14.10.2026**
(21) Application number: 24220547.4
(22) Date of filing: 17.12.2024
(51) Int. Cl.: A61N 1/04, A61N 1/39

(54) **SYSTEMS AND METHODS OF UTILIZING REMOVABLE DEFIBRILLATOR CARTRIDGES**
SYSTEME UND VERFAHREN ZUR VERWENDUNG ABNEHMBARER DEFIBRILLATORKARTUSCHEN
SYSTÈMES ET PROCÉDÉS D'UTILISATION DE CARTOUCHES DE DÉFIBRILLATEUR AMOVIBLES

(30) Priority: 28.12.2023 US 202363615461 P; 10.12.2024 AU 2024278239
(43) Date of publication of application: 02.07.2025
(73) Proprietor: Stryker Corporation, Portage, Michigan 49002 (US)
(72) Inventor: DONAGHY, Dymphna Mary, Portage, MI 49002 (US); ANDERSON, John Houston, Portage, MI 49002 (US)
(74) Representative: Kilburn & Strode LLP

(56) References cited:
- WO-A1-2018/109145
- US-A1- 2022 241 600
- US-B2- 11 229 801

## Description

### BACKGROUND

Example medical devices are configured to monitor and/or treat patients using complex techniques. In various cases, the medical devices are automated, manually operated, or a combination thereof. In some examples, the medical devices include automated external defibrillator (AEDs) with electrode pads. For instance, the electrode pads being placed on a patient enable the AEDS to identify electrical activity on the patient. In those or other cases, the electrical activity is analyzed to automatically identify an electrocardiogram (ECG) of the patient or to administer a defibrillation therapy to the patient. Documents US2022/241600A1, WO2018/109145A1 and US11,229,801B2 relate to AEDs.

### SUMMARY

The invention is defined by the independent claims. According to an aspect is provided a system, comprising a disposable cartridge, a sensor and an automated external defibrillator. The disposable cartridge can comprise: a battery; electrode pads configured to be disposed on a chest of a patient; and a memory storing an indication of a status of the disposable cartridge. The sensor can be configured to detect the status of the disposable cartridge, the status comprising a charge level of the battery or whether a charge has been received by the electrode pads. the automated external defibrillator (AED) can be configured to be powered by the disposable cartridge. The AED can comprise: a cavity configured to receive the disposable cartridge; a conductor configured to route, from the disposable cartridge, a notification signal indicating the status of the disposable cartridge; a measurement circuit configured to detect an electrocardiogram (ECG) of the patient; a treatment circuit configured to output an electrical shock; and a processor. The processor can be configured to: determine, by analyzing the status of the disposable cartridge, that the disposable cartridge is unused and ready to use; and in response to determining that the disposable cartridge is ready to use: cause the measurement circuit to be electrically coupled to the electrode pads in the disposable cartridge; cause the treatment circuit to be electrically coupled to the electrode pads in the disposable cartridge; and cause the treatment circuit to be electrically coupled to the battery in the disposable cartridge.

Optionally, the AED further comprises: a display configured to illuminate an indicator representing that the disposable cartridge is unused.

Optionally, the processor is further configured to: receive a communication from the disposable cartridge, in response to receiving the communication, analyze a flag with a default value representing the disposable cartridge being unused; and in response to analyzing the flag with the default value, control a display to switch to illuminating an indicator that symbolizes shocking the patient.

According to an aspect is provided a medical device, comprising: a memory; a cavity configured to receive a, e.g. disposable, cartridge that comprises electrode pads, a battery, and a storage device; a conductor configured to route a signal that identifies the cartridge is removably coupled to the cavity; a processor configured to analyze a signature communicated by the signal that matches a value stored in the memory, the signature being utilized to identify that the cartridge is unused; and a display configured to, in response to analyzing the signature, switch between illuminating any portion of the display to illuminating an indicator that symbolizes shocking a patient.

Optionally, the display is further configured to illuminate a use indicator according to a first mode (e.g. steady light, a first color, e.g. green, or the like) representing that the cartridge is unused. Optionally, the display is further configured to illuminate a use indicator according to the first mode representing that another cartridge being removably coupled to the cavity is unused. Optionally, the display is further configured to illuminate a use indicator according to a, different, second mode (e.g. blinking light, a second color, e.g. red, or the like) representing that the cartridge is used. Optionally, the display is further configured to illuminate a use indicator according to the second mode representing that another cartridge being removably coupled to the cavity is used.

Optionally, the display is further configured to illuminate a use indicator with a steady light representing that the cartridge is unused. Optionally, the display is further configured to illuminate a use indicator with a steady light representing that another cartridge being removably coupled to the cavity is unused. Optionally, the display is further configured to illuminate a use indicator with a blinking light representing that the cartridge is used. Optionally, the display is further configured to illuminate a use indicator with a blinking light representing that another cartridge being removably coupled to the cavity is used.

Optionally, the storage device comprises a memory card, read only memory (ROM), flash memory, a micro secure digital (SD) card, a SD card, a compact flash card, programmable read-only memory (PROM), electrically erasable, programmable, read-only memory (EEPROM), a smart card, a subscriber identity module (SIM) card, and/or a radio frequency identification (RFID) chip.

Optionally, the processor is further configured to: receive a communication from the disposable cartridge; in response to receiving the communication, analyze a flag with a default value representing the disposable cartridge being unused; and in response to analyzing the flag with the default value, control a display to switch to illuminating an indicator that symbolizes shocking the patient.

Optionally, the processor is further configured to: receive, from the cartridge, an identification signal indicating a serial number of the cartridge.

Optionally, a printed circuit board (PCB) is coupled between a first surface of the cartridge and a second surface of the cartridge, and an adhesive connects the first surface with the second surface by a seal.

Optionally, the medical device further comprises a transceiver, wherein the processor is further configured to: analyze an identifier received via a communication from the cartridge, the identifier being stored in another storage device of the cartridge and representing an age of the electrode pads or the battery; and in response to recognizing that the age is greater than a threshold, control the transceiver to transmit an alert.

Optionally, the processor is configured to: receive an identifier representing an age of the electrode pads or the battery of the cartridge; and in response to recognizing that the age is greater than a threshold, control presenting by the display of an alert.

Optionally, the medical device further comprises: a sensor configured to sense that a potential at a port of the cavity is modified in response to coupling the port with a pin of a connector of the cartridge, wherein analyzing the signature comprises analyzing the signature in response to the sensing that the potential is modified.

According to an aspect is provided a system, comprising: a disposable cartridge, a sensor and a medical device as described herein. The disposable cartridge can comprising: a battery; electrode pads configured to be disposed on a chest of a patient; and a storage device storing an indication of a status of the disposable cartridge. The sensor can be configured to detect the status of the disposable cartridge, the status comprising a charge level of the battery or whether a charge has been received by the electrode pads. The medical device can comprise an automated external defibrillator (AED) configured to be powered by the disposable cartridge, The AED can comprise: a measurement circuit and a treatment circuit. The measurement circuit can be configured to detect an electrocardiogram (ECG) of the patient. The treatment circuit can be configured to output an electrical shock. The processor can be further configured to: determine, by analyzing the status of the disposable cartridge, that the disposable cartridge is ready to use; and in response to determining that the disposable cartridge is ready to use: cause the measurement circuit to be electrically coupled to the electrode pads in the disposable cartridge; cause the treatment circuit to be electrically coupled to the electrode pads in the disposable cartridge; and cause the treatment circuit to be electrically coupled to the battery in the disposable cartridge.

According to an aspect is provided a method, comprising: receiving a signal that identifies a cartridge removably coupled to a cavity of a medical device, the cartridge comprising electrode pads, a battery, and a storage device; analyzing a signature communicated by the signal that matches a value stored in a memory of the medical device, the signature being utilized to identify that the cartridge is unused; and in response to analyzing the signature, controlling a display of the medical device to switch between illuminating any portion of the display to illuminating an indicator that symbolizes shocking a patient.

Optionally, the method further comprises: controlling the display to illuminate a use indicator with a steady light representing that the cartridge is unused.

Optionally, the method further comprises: controlling the display to illuminate a use indicator with a steady light representing that another cartridge being removably coupled to the cavity is unused.

Optionally, the method further comprises: controlling the display to illuminate a use indicator with a blinking light representing that the cartridge is used.

Optionally, the method further comprises: controlling the display to illuminate a use indicator with a blinking light representing that another cartridge being removably coupled to the cavity is used.

Optionally, the method further comprises: analyzing an identifier received via the signal that represents an age of the electrode pads or a battery; and in response to recognizing that the age is greater than a threshold, controlling a transceiver to transmit an alert.

Optionally, the method further comprises: transmitting a ping that requests a status of a characteristic of the cartridge being watertight; and receiving a response that confirms the characteristic is verified.

Optionally, the method further comprises: ceasing to receive the signal;
setting a flag corresponding to a status, the status representing the cartridge being unused, the status being linked to the value; receiving the signal; identifying that the flag is set in response to receiving the signal; and controlling the display to illuminate an alert in response to identifying that the flag is set.

Optionally, controlling the display further comprises: setting a flag identifying a first electrode pad that is positioned on a chest located on the patient and a second electrode pad that is positioned on a midsection located on the patient; identifying an electrocardiogram (ECG) characteristic in response to the setting of the flag; analyzing an amplitude represented by the ECG characteristic; confirming the amplitude is greater than a threshold; and controlling the display to illuminate the indicator in response to confirming the amplitude is greater than the threshold.

Optionally, controlling the display comprises setting the display to indicate that no mode is set; receiving a selection via input to the display that energizes utilizing the medical device for the patient that is a child; controlling the display to indicate placement of the electrode pads on the child; identifying a potential being changed that represents the electrode pads being positioned on the child; and controlling the display to switch to illuminating the indicator that symbolizes shocking the patient in response to identifying the potential being changed that represents the electrode pads being positioned on the child.

It will be appreciated that any of the aspects, features and options described herein can be combined. It will particularly be appreciated that any of the aspects, features and options described in view of the medical device apply equally to the systems and methods, and vice versa.

### BRIEF DESCRIPTION OF THE DRAWINGS

FIG. 1 illustrates an example environment in which a cartridge is removably couplable to an automated external defibrillator (AED) utilized to monitor and treat a condition of a patient.
FIG. 2 illustrates example cartridges and AEDs, the cartridges configured to be removably coupled to the AEDs, the cartridges including memory utilized to store usage data associated with the cartridges.
FIG. 3 illustrates example cartridges configured to be removably coupled to AEDs.
FIG. 4 illustrates an example AED that is non-adhesively sealed and that is connectable to a removably couplable cartridge.
FIG. 5 illustrates an example display of an AED, the display including a usage indicator associated with usage of a cartridge that is removably coupled to the AED.
FIGS. 6 and 7 illustrates example processes for utilizing a cartridge that is removably couplable to an AED utilized to monitor and treat a condition of a patient, the cartridge including memory utilized to store usage data associated with the cartridge.
FIG. 8 illustrates an example of an external defibrillator configured to perform various functions described herein.

### DETAILED DESCRIPTION

Various implementations described herein relate to disposable medical device cartridges that include disposable accessory devices for medical devices, and that are also capable of storage and communication of data indicative of a usage of the cartridges (referred herein as "usage data"). Accordingly, medical devices may be prevented from relying on expired and/or previously used cartridges. Various cartridges described herein improve medical device readiness by enabling medical devices to detect whether a connected accessory device should be replaced prior to use.

Medical devices, including automated external defibrillators (AEDs) are placed, maintained, or utilized in various types of environments for various lengths of time. Operational usability, reliability, understandability, and convenience of the AEDs is extremely important to enable rapid treatment of patients in emergency scenarios. Because AEDs are stored in varieties of locations, maintained according to varieties of types of schedules, procedures, and policies, and utilized in unexpected, unpredictable, and unforeseen circumstances, ensuring that sub-devices, portions, and/or components of the AEDs are in full operational condition can be crucial. However, it is difficult to ensure that AED accessories, which are often stored for long time periods (e.g., months or years), are ready for use in emergency situations.

In particular cases, AEDs utilize disposable accessories to perform functions (e.g., monitoring and treatment capabilities). However, if an AED is connected to a disposable accessory that has expired or been previously used, the AED may be unable to safely perform its functions. Thus, it is desirable for AEDs to detect whether accessories are ready to use.

Various implementations described herein address these and other problems by providing enhanced accessory-storing cartridges that report their readiness to external devices, such as medical devices (e.g., AEDs). For instance, cartridges can store and output usage data, which may indicate whether any of the accessory devices (e.g., batteries, electrode pads, disposable sensors, etc.) disposed in the cartridges have been previously used. In some cases, cartridges are configured to be removably coupled to the AEDs. For example, when a cartridge is coupled to an AED, a circuit in the cartridge is electrically coupled to a circuit in the AED. This connection, for instance, enables to cartridge to output data to the AED. This data may indicate readiness characteristics of the cartridge, such as whether the cartridge has been previously used, whether an accessory device in the cartridge has expired, and the like. Based on the data, the AED may determine whether the cartridge should be replaced prior to use. In various cases in which the cartridge is not ready for use, the AED may transmit, to an external device, an alert requesting that the cartridge can be replaced. In some examples, the AED audibly and/or visually indicates that the cartridge is expired. Accordingly, the AED may be outfitted with a new cartridge and may be ready for use in the case of a sudden medical emergency.

Implementations of the present disclosure are directed to improvements in the technical field of medical devices (e.g., AEDs). In particular examples of previous technologies, a used pair of electrode pads could be stored with an AED. If the AED is unable to identify that the electrode pads have been previously used, then the AED would not be able to report that the electrode pads should be replaced. In the case of a medical emergency in which a nearby individual collapses due to cardiac arrest, a bystander may attempt to use the AED with the previously used electrode pads. However, the previously used electrode pads would be unable to be used safely with the individual. For instance, an adhesive on the surfaces of the previously used electrode pads may be dried out and unable to attach the electrode pads to the skin of the individual's chest. As a result, the electrode pads may be inadequately electrically coupled to the individual. Thus, the AED would be unable to accurately detect an electrocardiogram (ECG) of the individual, thereby being unable to detect whether the individual has a shockable rhythm (e.g., ventricular fibrillation). Further, due to the poor electrical connection between the electrode pads and the individual's chest, the AED would be unable to adequately deliver an electrical shock that could defibrillate the individual. Such operational problems of the AED could have serious health consequences for the individual. In contrast, a cartridge described herein could address this problem by communicating, to the AED, whether the electrode pads have been previously used. Before the medical emergency, the AED could communicate that the cartridge should be replaced, and a maintenance person could replace the cartridge. Thus, implementations of the present disclosure could ensure that the electrode pads stored with the AED during the medical emergency are unused and ready to monitor and treat the individual.

Various examples will now be described with reference to the accompanying drawings.

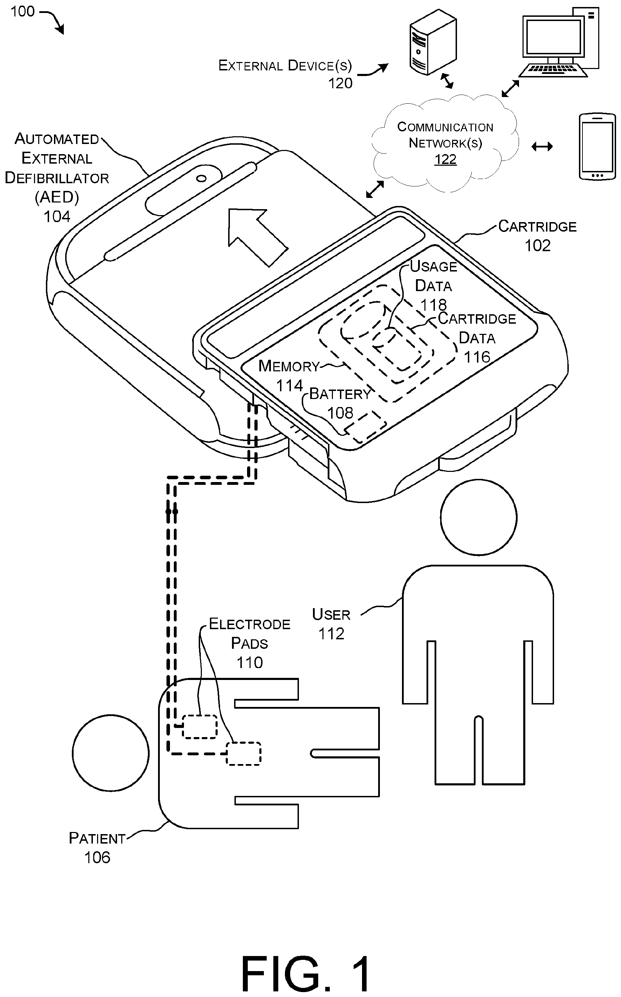

FIG. 1 illustrates an example environment 100 in which a cartridge 102 is removably couplable to an automated external defibrillator (AED) 104 utilized to monitor and treat a condition of a patient 106.

In various implementations, the example environment 100 includes a non-clinical setting. For example, the patient 106 may be experiencing a medical emergency outside of a clinical setting, such as a hospital. In particular instances, the patient 106 may have suddenly collapsed in a public area, such as a school, airport terminal, or office building.

In some cases, the patient 106 is experiencing sudden cardiac arrest. For example, the patient 106 may have a cardiac arrhythmia that prevents the heart of the patient 106 from effectively pumping blood through the body of the patient 106. Examples of such cardiac arrhythmias include ventricular fibrillation (VF) and pulseless ventricular tachycardia (VT). As a result of the lack of oxygenated blood flow through the body of the patient 106, various tissues in the body of the patient 106 may eventually experience a hypoxic injury. In particular cases, a sustained lack of blood flow can cause damage to the brain and other vital organs of the patient 106.

To avoid significant and permanent damage to the patient 106, the AED 104 may be configured to monitor a condition of the patient 106 and/or administer a treatment that monitors and/or treats the medical emergency of the patient 106. For example, the AED 104 is configured to detect an electrocardiogram (ECG) of the patient 106. The AED 104 analyzes the ECG. In some examples, if the AED 104 detects that the ECG is indicative of a shockable arrhythmia (e.g., VF or pulseless VT), the AED 104 may recommend that the patient 106 is treated with an electrical shock. In some examples, the AED 104 may output a notification and/or indication to recommend the treatment with the electrical shock. In those or other examples, the AED 104 automatically prepares and discharges the electrical shock in response to detecting the shockable arrhythmia.

The cartridge 102 includes one or more devices that can be used by the AED 104 to monitor and/or treat the patient 106. In some cases, the cartridge 102 includes a power source (or "power supply") (e.g., a battery) 108 that is configured to power the AED 104. In some cases, the cartridge 102 includes a processor (e.g., an application-specific integrated circuit (ASIC), or any other type of processor) that is configured to perform any of one or more functions of the cartridge 102, as discussed herein. In some examples, the cartridge 102 includes one or more accessory devices. For example, the cartridge 102 includes a pair of electrode pads 110 configured to be coupled to the patient 106 during use of the AED 104. The AED 104, for instance, is configured to detect the ECG of the patient 106 using the accessory device. In some cases, the AED 104 is also configured to discharge the electrical shock via the electrode pads 110.

Other types of accessory devices may be stored in the cartridge 102. Examples of accessory devices include, for instance, electrode pads, physiological sensors (e.g., a blood pressure cuff, a pulse oximeter, a capnography sensor, a pulse sensor, etc.), a pump configured to administer a medication subcutaneously or intravenously, a band configured to administer chest compressions, a plunger configured to administer chest compressions, an accelerometer, a pressure sensor, a chest compression detector, or any combination thereof. In various cases, the accessory device(s) are single-use and/or disposable. For instance, the accessory device(s) may not function appropriately if they are used to monitor and/or treat more than one patient.

In various implementations, the cartridge 102 is physically and electrically coupled to the AED 104. In various cases, a circuit in the cartridge 102 is electrically coupled to a circuit in the AED 104 when the cartridge 102 is physically coupled to the AED 104. For instance, the battery and/or accessory device(s) in the cartridge 102 are physically and electrically coupled to the AED 104 when the cartridge 102 is attached to the AED 104. The cartridge 102 may be removed from the AED 104, such that the cartridge 102 can be replaced (e.g., by another cartridge).

In some cases, the cartridge 102 includes one or more components (e.g., one or more sub-devices). The components, for instance, include the accessory device(s) and/or additional elements. For example, the component(s) (e.g., the sub-device(s)) of the cartridge 102 include electrode pads (e.g., the electrode pads 110), one or more power supply devices (e.g., the battery 108), one or more processors (e.g., the ASIC), one or more sensors, one or more visual indicator devices, one or more converters (e.g., one or more digital to analog converters (DAC(s)), one or more speakers, one or more microphones, one or more user interfaces (Ul(s)), one or more mechanisms (e.g., one or more user input mechanisms) (e.g., one or more buttons, one or more switches, one or more sliders, one or more knobs, one or more of any other types of mechanisms, or any combination thereof), various types of other sub-devices, or any combination thereof.

According to some examples, the cartridge 102 and the AED 104 are stored in the non-clinical setting. Thus, the cartridge 102 and the AED 104 may be used to provide rapid care to the patient 106 before the patient 106 is transported to a clinical setting, such as a hospital.

In order to administer care to the patient 106 in the non-clinical setting, the AED 104 and the cartridge 102, are configured to be used by bystanders with limited to no medical training. A user 112 is operating the AED 104, in various examples. For instance, the user 112 is an untrained user. In some cases, the user 112 may be an individual in the non-clinical setting who happened to be present when the patient 106 began to experience the medical emergency. In some cases, the user 112 has removed the AED 104 (e.g., coupled to the cartridge 102) from one or more cabinets or one or more other storage mechanisms disposed in the non-clinical setting, and has brought the AED 104 to the side of the patient 106. According to some examples, the cartridge 102 and AED 104 are portable.

The AED 104, for instance, provides instructions to the user 112 for operating the AED 104. Accordingly, the user 112 may utilize the AED 104 to administer a complex treatment to the patient 106 despite their lack of specialized medical care. In some cases, a particular order and/or timing of instructions are presented by the AED 104. The order and/or timing of instructions, for instance, may be triggered based on a detected condition of the patient 106. In some cases, the order and/or timing of the instructions is triggered based on an operation of the AED 104 by the user 112. According to various examples, the AED 104 outputs (e.g., communicates and/or visually/audibly outputs) data and/or instructions (e.g., communicated instructions, and/or visual and/or audible instructions) to the user 112 that guide the use of the AED 104.

In various implementations, the cartridge 102 includes memory 114 utilized to store cartridge data 116 associated with the cartridge 102. In various implementations, the memory 114 stores various types of data associated with the cartridge 102. For example, the cartridge data 116 includes usage data 118 that indicates whether the accessory device(s), or other components stored in the cartridge 102, have been previously used. In some cases, the cartridge data 116 includes an identifier of the cartridge 102, such as a model number, a manufacturer, or an identification number that is uniquely associated with the cartridge 102. Other information can also be included in the cartridge data 116. For instance, the cartridge data 116 may include an expiration date of the cartridge 102.

In some instances, the cartridge 102 performs one or more operations on the cartridge data 116 stored in the memory 114. For example, the processor(s) of the cartridge 102 may be configured to perform the operation(s). Examples of the operations include generating, storing, modifying, and communicating the data. According to some examples, the cartridge 102 includes a circuit that detects whether the accessory device(s) (e.g., the electrode pads 110 and the battery 108) have been previously used. In some examples, the cartridge 102 includes a sensor configured to detect one or more characteristics associated with the electrode pads 110. For instance, the characteristic(s) associated with the electrode pads 110 include whether the electrode pads 110 have been previously removed from packaging, whether packaging disposed around the electrode pads 110 during storage has been breached, whether a cover of an adhesive disposed on the electrode pads 110 has been removed, or whether an electrical shock has been previously discharged to the electrode pads 110. According to some examples, the cartridge 102 includes a sensor configured to detect whether a charge level of the battery 108 is below a threshold charge level. In various cases, the cartridge 102 is configured to write and/or modify the usage data 118 in response to detecting that the electrode pads 110 and/or the battery 108 have been used.

In various implementations, the cartridge 102 is utilized to manage various types of data for various purposes and at various times during the life cycle of the cartridge 102. Some types of data that is included in the cartridge data 116 and that is stored before the cartridge 102 is utilized include, for example, types of data that enable the user(s) to identify that the cartridge 102 is the correct type of cartridge. In some cases, some permanently stored data for such purposes includes part numbers of the cartridge 102 or any of its components, compatibility data, such as identifiers (e.g., a product identifier, such as a product number associated with an AED) of types of AEDs with which the cartridge 102 is compatible, or other types of data.

In some examples, the cartridge data 116 provides crucial data to the AED 104, enabling the user(s) to easily and accurately verify that the cartridge 102 will operate correctly when needed, in addition to whether the cartridge is charged (e.g., has a fully charged power supply) and unused (e.g., has unused electrode pads). The cartridge data 116 may be utilized by the AED 104 to check that the cartridge 102 is a verified cartridge, such as a cartridge with a verified product identifier, manufacturer identifier, and/or any other type of identifiers. By using the cartridge data 116, the AED 104 is able to determine whether to provide an indicator that the cartridge 102 is ready to be used, such as a charged and new indicator, or to provide an indicator that a cartridge in an AED is not ready to be used and/or that a cartridge in an AED may not operate properly.

In those or other examples, providing quick and easy to understand information to the user 112 enables the user 112 to quickly identify whether the AED 104 is usable and ready for providing medical treatment to a patient, or somehow not usable or not ready. Various types of indicators are possible, such as a warning that a cartridge is able to used, but is not verifiable. For example, if a cartridge does not have a verifiable product identifier and/or a verifiable manufacturer identifier the user 112 may decide to use an AED if no other AEDs or other medical devices are available. However, the user 112 may be able to make a decision to use other verifiable equipment (e.g., another verified AED) quickly and easily, based on indicators provided by the AED 104 using the cartridge data 116. Such circumstance in which the user 112 is able to obtain accurate and crucial information may enable the user to successfully treat a patient and save the patient's life.

In various examples, the cartridge data 116 includes data stored at startup and/or at the beginning of the life cycle of the cartridge 102. In some instances, the data stored at the beginning of the life cycle includes data within the cartridge data 116 and/or including data within the usage data 118. For example, the data stored in the usage data 118 at the beginning of the life cycle includes data indicating that the cartridge 102 is new and unused. In various cases, after the user 112 uses the cartridge 102, the data indicating that the cartridge 102 is new and unused is updated.

In some implementations, the AED 104 automatically updates the cartridge data 116 (e.g., transmits one or more update signals to the cartridge 102) at a beginning of an occurrence in which the cartridge 102 is being used (e.g., at the beginning of any cartridge use(s) and/or operation(s) of any type(s)). Alternatively or additionally, the cartridge 102 and/or the AED 104 automatically update the cartridge data 116 during operation of the cartridge 102, such as at some time and/or while some process is occurring.

For instance, the AED 104 may be operated by the user 112, based on the user 112 being in a vicinity in an environment of the patient 106. The user 112 may obtain the AED 104 from where the AED is stored and/or kept. In some examples, the cartridge 102 and/or the AED 104 may utilize any of the sensor(s) (e.g., the gyroscope, the accelerometer, the touch sensor, etc.) to identify contact, motion, etc., and/or any other aspects associated with activity of the cartridge 102, the AED 104, and/or the user 112. The cartridge 102 and/or AED 104 may update the cartridge data 116 (e.g., perform one or more update operations for the usage data 118 and/or other data in the cartridge data 116) based on the activity being sensed, and/or any sensor data, associated with the sensor(s) of the cartridge 102 and/or the AED 104.

The user 112 may provide one or more selections via user input to the AED 104 (e.g., the user 112 may press a button) and/or extend the electrode pads 110 from the cartridge 102. Based on cartridge 102 and/or the AED 104 sensing (e.g., via the sensor(s)) any movement (e.g., extension) of the electrode pads 110, any electrical usage of the cartridge 102 (e.g., the battery 108), one or more of the electrode pads 110 being positioned correctly on the patient, and/or one or more types of other activity associated with operation of the cartridge 102 and/or the AED beginning, the cartridge 102 and/or AED 104 may update the cartridge data 116 (e.g., perform one or more update operations for the usage data 118 and/or other data in the cartridge data 116).

In those or other examples, the AED 104 automatically updates the usage data 118 and/or other data in the cartridge data 116 following operation the AED 104 utilizing the cartridge 102, such as at some completion time of any operation and/or process (e.g., an end of the operation and/or process, or after the operation and/or process ceases) and/or after all processes cease. In some instances, based on cartridge 102 and/or the AED 104 sensing (e.g., via the sensor(s)), a time that is identified after expiration of a threshold period of time during which there is a lack of any movement (e.g., extension) of the electrode pads 110 may be identified as the completion time. In those or other instances, a time that is identified after expiration of a threshold period of time during which there is no electrical usage of the cartridge 102 (e.g., the battery 108) may be identified as the completion time. In In those or other instances, a time that is identified after expiration of a threshold period of time during which there is a lack of one or more types of other activity associated with operation of the AED 104 with the cartridge 102, the cartridge 102 and/or AED 104 may be identified as the completion time.

In various examples, the AED 104 may identify usage being completed based on one or more of the electrode pads 110 being positioned correctly on the patient. In alternative or additional examples, the AED 104 identifies usage being completed based on data associated with the patient 106 being sensed (e.g., sensed via the electrode pads 110). In alternative or additional examples, the AED 104 identifies usage being completed based on electrical shocks being administered (e.g., administered via the electrode pads 110) to the patient 106. In alternative or additional examples, the AED 104 identifies usage being completed based on a charge level of the battery 108 falling below a threshold charge level. In alternative or additional examples, the AED 104 identifies usage being completed based on one or more identifiers (e.g., patient status identifiers) indicating patient activity (e.g., physiological data) is normal and/or that the medical emergency is over due to successful treatment of the patient 106. In alternative or additional examples, the AED 104 identifies usage being completed based on one or more other aspects of treatment associated with the cartridge 102, the AED 104, and/or the patient 106 being completed, or any combination thereof. In various cases, any of the aspects associated with the cartridge 102, the AED 104, and/or the patient 106 being completed may be utilized to update the cartridge data 116 (e.g., perform one or more update operations for the cartridge data 116).

In some cases, such as with the cartridge 102 being utilized to store different types of data of various types at various times, manipulation of some portions (e.g., one or more hardware portions and/or one or more software subsets) of the cartridge data 116 is enabled. In various examples, manipulation of other portions (e.g., one or more other hardware portions and/or one or more other software subsets) of the cartridge data 116 is not enabled. In various instances, various rules, capabilities, and/or restrictions associated with various subsets and/or various types of the cartridge data 116 enable the cartridge 102 to be utilized optimally and safely. In some cases, one or more subsets of the cartridge data 116 are initially stored in the cartridge 102 when the cartridge 102 is manufactured. For instance, any of the subset(s) of the cartridge data being initially stored is utilized to enable initial usage of the cartridge 102. For instance, the subset(s) of the cartridge data being initially stored are stored in the cartridge 102 during various processes prior to any utilization of the cartridge 102 by any user. In such an instance or another instance, the subset(s) of the cartridge data being initially stored are stored in the memory 114.

In various instances, the subset(s) of the cartridge data 116 being initially stored in the cartridge 102 include a partial set of the cartridge data 116. In some cases, the partial set being initially stored is the same as, or different from, the partial set at the end of the life of the cartridge 102. For example, the partial set of the cartridge data of which the entire amount that is initially stored is the same as the partial set at the end of the life of the cartridge 102, includes permanent, unalterable, data (e.g., data hard-wired into the cartridge 102, and/or data stored and/or managed with a read-only permission). In some examples, any of the hardware and/or software of the cartridge 102 and/or the AED 104 may be utilized to prevent the subset(s) of the cartridge data 116 being managed as the unalterable data from being altered.

In various cases, the unalterable data includes data in the cartridge data 116 that is managed separately, and differently, from the usage data 118. By managing the unalterable data separately, and differently, from the usage data 118, the cartridge 102 is able to reliably maintain and/or manage the usage data 118.

In some implementations, the subset(s) of the component data being stored as unalterable data are especially important to operation of the cartridge 102. In some cases, the subset(s) of the component data being stored as unalterable data include one or more groups of identifier data, one or more groups of date data, one or more groups of various other types of component data, or any combination thereof. The identifier data and the date data include one or more identifiers and one or more dates, respectively, associated with the sub-devices of the cartridge 102, for example, as discussed below in further detail. In some examples, the unalterable data includes one or more signatures in the cartridge data identifying the cartridge 102, and, possibly, identifying the sub-devices of the cartridge 102. For instance, the signatures identify the electrode pads 110 and/or the battery 108.

In some examples, one or more permissions (e.g., the read-only permission) are utilized to control access, and/or modification rights, associated with the partial set of the cartridge data 116. By restricting operations of the cartridge 102 that would otherwise be utilized to modify the partial set of the cartridge data 116, the partial set of the cartridge data 116 is protected. By protecting the partial set of the cartridge data 116, the cartridge 102 is secured from unwanted tampering, unexpected alterations, and/or incidental operations, so that the cartridge 102 is verifiable as a correct type of cartridge, a compatible cartridge, a cartridge that meets one or more standards of manufacturing, production, design, maintenance, etc. By ensuring that important data is not tampered with and/or changed, the likelihood of the user 112 being able to medically treat the patient 106 successfully and confidently is improved.

In various instances, the cartridge data 116 includes one or more subsets of component data (e.g., data which is associated with the cartridge 102, the electrode pads 110, the battery 108, any other components (e.g., other sub-devices, hardware, etc. of the cartridge 102), or any combination thereof). For example, the subset(s) of component data are stored as the unalterable data. In those or other instances, the cartridge 102 is designed to include, for example, hardware and/or software that prevents modification of any of the component data. In those or other instances, the hardware and/or software preventing modification to the component data is utilized to ensure that the unalterable component data is not modified, restrict modifications to the unalterable component data, render the unalterable component data incapable of being modified, or any combination thereof.

According to various implementations, the cartridge 102 is configured to perform additional operations based on the cartridge data 116. In some cases, the cartridge 102 is configured to output, to the AED 104, a communication signal based on the usage data 118 and/or other data in the cartridge data 116. For instance, the cartridge 102 may output, to the AED 104, a signal indicating that the cartridge 102 is unused and ready for operation, or a signal indicating that the cartridge 102 has been previously used and is not ready for operation, depending on the usage data 118. In some examples, the cartridge 102 outputs an instruction to replace the cartridge 102, upon determining that the cartridge 102 has been previously used.

In some cases, the AED 104 is configured to transmit, to one or more external devices 120, such as via one or more communication networks 122, a communication signal based on the signal from the cartridge 102. For example, the AED 104 may wirelessly transmit an indication that the cartridge 102 should be replaced. In various implementations, the AED 104 is utilized (e.g., managed, automated, controlled, etc., or any combination thereof) to exchange one or more communications with the external device(s) 120 (e.g., external device(s) of any of one or more users). The user(s), for instance, include one or more users of various types, such as facility personnel, maintenance personnel, medical personnel, any other personnel of various types, or any combination thereof). In some examples, the AED 104 transmits the subset(s) of the cartridge data 116 to the external device(s) 120 at startup and/or at a beginning of a life cycle and/or a usage, during operation, and/or at completion of operation, as discussed below in further detail. In those or other examples, the AED 104 additionally transmits an identifier of the AED 104, such as a location of the AED 104 or an alphanumeric identification code that is uniquely associated with the AED 104. Thus, if an external device 120 is communicatively coupled to multiple devices including the AED 104, then the external device 120 may be able to identify the AED 104 that is connected to an inadequate cartridge. In some cases, the external device 120 outputs a notification to a maintenance user (not illustrated). Upon receiving the notification, the AED 104 may cause the maintenance user to physically decouple the expired cartridge from the AED 104 and replace it with a new cartridge. According to various cases, one or more cartridges may be replaced prior to the medical emergency in FIG. 1, such that the user 112 may operate the AED 104 with the cartridge 102 rapidly in response to the sudden medical emergency of the patient 106.

In some examples, the AED 104 outputs a signal to the user 112 based on the signal received from the cartridge 102. For example, the AED 104 may output, via a user interface, an instruction to replace the cartridge 102 or a notification that the cartridge 102 is ready to use. By verifying that the AED 104 is fully functional, with a full charge in the battery 108 and with the electrode pads 110, the user 112 has a significantly higher likelihood of successfully administering the medical treatment with the AED 104 and to the patient 106. Such beneficial functionality of the AED 104 and the cartridge 102 increases the likelihood, in some scenarios, of the user 112 being able to medically treat the patient 106 successfully, and/or to save the life of the patient 106.

In some examples, various types of crucial data are obtainable, from the AED 104 and via the cartridge data 116. For example, various types of crucial data are obtainable by the user 112, such as whether the battery 108 is fully charged and/or whether the electrode pads 110 are new and/or unused. Such information is extremely important, and any omission of providing information to the user 112 could potentially result in various disastrous consequences. For example, if the user 112 is unable to obtain a level of charge of the battery 108, and if the battery 108 is depleted, the AED 104 may fail to appropriately administer a treatment to the patient 106, and the patient 106 could die without treatment. If the electrode pads 110 have been previously used, for example, then they would not stick to the patient 106 effectively, and the AED 104 would not be able to detect an ECG of the patient 106 accurately or administer an appropriate shock. Implementations of the present disclosure can be used to prevent these potential problems.

By managing and reporting the cartridge data 116, the cartridge 102 enables the user 112 to verify that the cartridge 102 will operate satisfactorily and successful before beginning to treat the patient 106. In contrast to AEDs implemented according to conventional technology, which do not include removable cartridges that store cartridge data, including usage data 118, the user 112 is able to utilize the AED 104 with the cartridge 102 to handle medical emergencies safely, quickly, reliably, and confidently.

In some examples, the usage data 118 enhances proper usage of the AED 104. For instance, the user 112 obtains information indicating that the AED 104 is operationally ready, based on the battery 108 being fully charged and the electrode pads 110 being new. In some cases, the user 112, obtaining information indicating that the AED 104 is operationally ready, is able to utilize the AED 104 confidently for treating the patient 106. In some cases, the usage data 118 is utilized to report to the user 112 the operational readiness of the AED 104, such as a ready indicator being illuminated based on the fully charged level of the battery 108 and the new condition of the electrode pads 110. The usage data 118 being utilized by the AED 104 to report to the user 112 the operational readiness of the AED 104, enables treatment to be successfully provided to the patient 106, thereby event potentially saving the life of the patient 106 in some cases.

In various cases, communication (e.g., transmission via the transceiver(s)) being performed by the AED 104 includes the AED 104 transmitting, to the external device(s) 120, the subset(s) of the cartridge data 116 in various ways. For example, any of the subset(s) of the cartridge data 116 are transmitted to the external device(s) 120 using one or more identifiers. The identifier(s) include, for instance, one or more prestored identifiers, one or more user configured identifiers, one or more identifiers of other types, or any combination thereof.

By utilizing the AED 104 to perform the automated communication(s), the user(s), are notified of any of one or more aspects and/or conditions associated with the cartridge 102, any components therein, the AED 104, and/or any components therein. For instance, the condition(s) include one or more updates, one or more changes, one or more warnings, one or more completions of life expectancies, one or more alerts, and/or one or more of other types of conditions. In some cases, the automated communication(s) are exchanged to distribute the information associated with the cartridge 102 and/or the AED 104 to enable the cartridge 102 and/or the AED 104 to be surveyed, maintained, tracked, inventoried, scanned, etc., in a remote, predictable, scheduled, and/or reliable way.

In various examples, the AED 104 outputs (e.g., transmits and/or presents) at least one of the usage data notification(s) at various times, based on at least one communication from the cartridge 102. In some cases, the usage data notification(s) include one or more corresponding alerts. For example, the AED 104 outputs an alert in response to the cartridge 102 indicating, based on the usage data 118, that at least one component of the cartridge 102 has been previously used.

For instance, the AED 104 may prepare a signature (e.g., a cartridge signature of the cartridge) in the cartridge data 116 with a cartridge signature in the data stored in the AED 104 to identify a usage value (or "usage status" or "status"). In some cases, the usage value is stored in the cartridge 102 and/or the AED 104, and is linked to the cartridge signature. In those or other cases, the usage data notification(s) are output (e.g., transmitted and/or visually/audibly output) to indicate, based on the usage value and via a usage identifier indicating that the cartridge 102 has been used "0" times and that the cartridge 102 is usable. For instance, the usage data notification(s) are transmitted and/or visually/audibly output to indicate the usage identifier having a value of "0," by illuminating one or more indicators. In some examples, the display of the AED 104 switches from illuminating any of one or more other portions (e.g., one or more other indicators) of the display to illuminating the usage identifier. In various cases, the usage value stored in the AED 104 is managed according to, and/or linked with, the usage value stored in the cartridge 102.

Although the AED 104 may output (e.g., transmits and/or visually/audibly outputs) the usage data notification(s) in various ways, as discussed above in the current disclosure, it is not limited as such. In some examples, the AED 104 performs normal operations based on the usage data 118 indicating that the cartridge 102 is new, and/or that the sub-devices in the cartridge 102 is new. In those or other examples, the AED 104 automatically proceeds with performing operations at various times based on the usage data 118 indicating that the cartridge 102 is new, and/or that the components in the cartridge 102 is new. In some cases, the AED 104 automatically proceeds with performing operations because any operability and/or safety issues/concerns are minimized in such scenarios, thereby. For instance, such as with the cartridge 102 being new, a usage value (or "initial value") (or "default value") initially stored in the usage data 118 represents a usage of "0" times. In some examples, the usage value is "0," new, any other value representing the cartridge 102 being new and/or unused, or any combination thereof.

In some examples, one or more notifications are output to indicate that at least one of the portion(s) of the cartridge 102 and/or at least one of the portion(s) of the AED 104 are incompatible, unsatisfactory, or any combination thereof. For example, one or more notifications are transmitted by one or more transceivers, presented by one or more displays, one or more audible notifications being output by one or more speakers, any other types of output, or any combination thereof.

In some cases, various types of incompatibility are identified in the notification(s). For example, component and/or software incompatibility is identified in the notification(s) based on modified, changed, and/or updated component data that does not match initially stored component data. In those or other cases, the notification(s) being output indicate any of the modified, changed, and/or updated component data that does not match the initially stored component data. For instance, the non-matching component data may be associated with one or more different components from the initial component(s).

In various cases, the identification, such as via the notification(s), of the incompatible and/or the unsatisfactory portion(s) is automated. For example, the various types of incompatibility are identified based on status data not matching corresponding initial status data. In some cases, the status data includes data that is part of the cartridge data 116 and that indicates any existence of the modified, changed, and/or updated component data. In some cases, the various types of incompatibility are identified based on the status data and/or one or more serial numbers. For instance, the serial number(s) are included in the new component data associated with the cartridge 102 and/or corresponding sub-device(s).

In some cases, the status data indicates that individual ones of the serial number(s) in the component data do not match corresponding initial serial numbers (e.g., corresponding valid serial numbers). In alternative or additional cases, the various types of incompatibility are identified via the status data. For instance, the incompatibility is identified based on one or more dates in the component data associated with a cartridge and/or corresponding sub-device(s) being manufactured, shipped, authorized, etc., not matching one or more corresponding initial dates associated with the cartridge 102 and/or corresponding sub-device(s) being manufactured, shipped, authorized, etc.

In some cases, a problem is identified based on new component data indicating that a replacement cartridge inserted in the AED 104 is a wrong type and/or is incompatible with the AED 104. The status data, for example, identifies and is utilized to report out, via the communication(s), the problem to the external device(s) 120.

Although the cartridge 102 may be removably couplable to the AED 104 for various purposes, as discussed above in the current disclosure, it is not limited as such. In some examples, any of one or more different cartridges (e.g., a new, fresh, cartridge) is utilized by the AED 104 in a similar way as for the cartridge 102, for example, for purposes of implementing any of the techniques as discussed throughout this disclosure.

Although various types of data crucial to ensuring safe operation of the cartridge 102 and/or the AED 104 may be utilized, as discussed above in the current disclosure, it is not limited as such. In some examples, other types of the data, which are relatively less important, are excluded (e.g., such as, based on settings data) from being utilizable to control the cartridge 102. In such an example, the relatively less important types of the data may be utilized, for instance, to inform the user(s) that some portions of the cartridge, such as an indicator utilized to illuminate as light provided for convenience of operating and/or location the cartridge 102 and/or the AED 104, is expired and/or broken.

Although the cartridge data 116 may include various types of status data, as discussed above in the current disclosure, it is not limited as such. In some examples, the status data includes one or more airtight statuses associated with whether the cartridge is airtight. In such an example or another example, the cartridge 102 includes one or more sensors, including vacuum sensors, air pressure sensors, air flow sensors, air quality sensors (e.g., sensors to measure levels of various gases), any other types of air sensors, any of other types of sensors., or any combination thereof. In some examples, the sensors identify (e.g., detect) one or more base values (e.g., one or more base air pressure values, and/or one or more other base values/metrics associated with characteristics of an interior of the cartridge 102). For instance, the base pressure air value(s) include initial air pressure values detected at initial activation of the cartridge 102, one or more thresholds, one or more other values of various types, or any combination thereof.

In various examples, the cartridge 102 utilizes the sensor(s) to identify (e.g., detect) one or more metrics and/or one or more values. The metric(s)/value(s) include, for instance, one or more air pressure values, and/or one or more other metric(s)/value(s) associated with characteristics of an interior of the cartridge 102. In those or other examples, the cartridge 102 identifies one or more status values of the status data based on one or more corresponding comparisons of whether the detected value(s) match, and/or are within threshold differences from, the base value(s). For instance, the status value(s) may indicate whether the detected air pressure value(s) match, and/or are within threshold differences from, the base air pressure value(s).

Although the cartridge 102 may include the various types of sensor(s), as discussed above in the current disclosure, it is not limited as such. In various examples, the cartridge 102 includes one or more sensors utilized to identify data associated with any of the sub-device(s) of the cartridge 102. For example, the cartridge 102 includes one or more pressure sensors, one or more electromagnetic sensors, one or more magnetic sensors, one or more light sensors (e.g., one or more infrared light sensors, one or more visible light sensors, one or more of any types of light sensors), one or more movement sensors associated with any components and/or portions of the frame and/or the housing of the cartridge 102, etc., and/or one or more sensors of any other types. In various examples, the sensors include one or more battery characteristics sensors. For instance, the battery characteristics sensor(s) include one or more voltage sensors, current sensors, and/or charge state sensors, any other battery related sensors, or any combination thereof.

In some instances, the cartridge 102 utilizes the sensor(s) to obtain data associated with the electrode pads 110, such as data indicating the electrode pads 110 and/or any portion of the cartridge frame and/or housing are accessed by the user 112 (e.g., touched, opened, moved, etc.). In those or other instances, the cartridge 102 utilizes the sensor(s) to obtain data associated with the battery 108, such as data indicating the battery 108 and/or any portion of the cartridge frame and/or housing are accessed by the user 112 (e.g., touched, opened, moved, etc.), and/or whether any level of charge of the battery 108 changes, etc., or any combination thereof..

Although the component data may include the identifier data, the date data, the various other types of component data, or any combination thereof, as discussed above in the current disclosure, it is not limited as such. In some examples, the component data includes characteristics data associated with the cartridge 102 and/or the sub-device(s) therein. In various cases, any subsets of the characteristics data associated with the cartridge 102 and/or the AED 104, and/or with any of the sub-device(s) therein, being altered. In those or other cases, any subsets of the characteristics data are associated with the cartridge 102 and/or the AED 104, and/or with any of the sub-device(s) therein, being modified, enhanced, expanded, redacted, or altered. In those or other cases, any subsets of the characteristics data are based on any changes to the sub-device(s) (e.g., the cartridge 102, the electrode pads 110, and/or the battery 108, and/or any other of the sub-device(s) being swapped out, etc.). In those or other cases, any subsets of the characteristics data are utilized in a similar way as any of the cartridge data 116 for purposes of implementing any of the techniques discussed herein. In some cases, the characteristic(s) are associated with, and/or utilized to identify, the detected value(s) (e.g., the detected air pressure value(s)) and/or the metric(s).

Although the AED 104 may be configured to exchange communications with the external device(s) 120, as discussed above in the current disclosure, it is not limited as such. In various cases, any of the communication(s) are performed at one or more of various times, based on one or more occurrences of various types. For example, any of the communication(s) are performed during swap-outs/replacements of the cartridge 102 and/or the sub-device(s). The AED 104 includes, in some cases, transceivers, readers, etc., usable for storing any of the cartridge data 116, and/or for communicating with any of the external device(s) 120. For instance, the AED 104 includes one or more of any types of devices, such as universal product codes (UPC), quick response (QR) codes, radio frequency identification (RFID) tag devices (e.g., transceivers), near field communication (NFC) devices (e.g., transceivers), etc.

Although the cartridge 102 may be configured to perform any of the various operations based on the cartridge 102 having been previously used, as discussed above in the current disclosure, it is not limited as such. In some cases, the cartridge 102 includes a circuit that automatically blocks an electrical signal between the AED 104 and any accessory or component in the cartridge 102 in response to determining that the cartridge 102 has been previously used. For example, due to the cartridge 102 having been previously used, the cartridge 102 may prevent the AED 104 from receiving a signal indicative of the ECG of the patient 106 from the electrode pads 110. For example, alternatively or additionally, due to the cartridge 102 having been previously used, the cartridge 102 may prevent an electrical shock from being discharged to the patient 106 via the electrode pads 110. For example, alternatively or additionally, due to the cartridge 102 having been previously used, the cartridge 102 may prevent the battery 108 from supplying electrical energy to the AED 104

Although the cartridge data may include unalterable data, as discussed above in the current disclosure, it is not limited as such. In various examples, the cartridge data 116 includes restricted data. For instance, one or more of the subset(s) of the unalterable data and/or any other subsets of the cartridge data 116 include restricted data that is not enabled for communication to any device but the AED 104. By restricting communication (e.g., transmission) of the restricted data, security of the AED 104 and/or the cartridge 102 is increased.

In some cases, the restricted data includes any data unique to the cartridge 102. For example, the restricted data includes the identifier(s) that are unique for the cartridge 102, and/or one or more portions of the cartridge data 116 that are not able to be communicated for security protection of the cartridge 102 and/or the AED 104, and/or that are not usable and/or of interest to one or more users of the external device(s) 120. In some cases, the restricted data includes fully restricted data that is restricted from transfer to other external devices.

By way of example, utilization of the AED 104 includes the AED 104 being located in a library. For instance, the patient 106 may be a customer or member this is visiting a library and that collapses. In such an example, the user 112 may be another customer or, perhaps, a librarian that witnesses the patient 106 collapsing or that comes across the patient 106 lying on the floor or sitting on a chair and leaning on a desk, after collapsing. The user 112, for example, obtains the AED 104 from a location in which the AED 104 is kept, such as a storage location in the library. In some instances, the AED 104 is kept on a wall in an enclosure mounted on the wall for purposes of holding the AED 104.

In various example, because the AED 104 communicates the usage data 118 to a technician device, a technician maintains the cartridge 102 prior to usage of the AED 104 by the user 112. The technician, for instance, makes sure that the cartridge 102 is new and unused. The cartridge 102, in some instances, may include an original cartridge included in the AED 104 or a replacement cartridge removable inserted in the AED 104 after one or more other cartridges were used, expired, had depleted battery level(s), or any combination thereof. In various cases, the AED 104 transmits a message to the technician device and is serviced by the technician based on the technician viewing the message. For example, the technician places the cartridge 102 with the full battery 108 and unused electrode pads 110 in the AED 104. In some implementations, the AED 104 outputs (e.g., visually by a screen, audibly by a speaker, or the like) an indication of the state of the cartridge 102 (e.g., whether the cartridge 102 is expired). In some cases, the AED 104 transmits, to an external device (e.g., a server), an indication of the state of the cartridge 102. In case in which the AED 104 is a part of a fleet of devices, the external device may output a report indicating which devices within the fleet are in need of replacement cartridges (e.g., including the cartridge 102). Thus, a technician can efficiently manage the fleet of devices.

In various instances, the user 112 lifts up the AED 104, which includes the cartridge 102, carries the AED 104, and places the AED 104 next to the patient 106. In various examples, the user 112 is able to quickly and easily identify that the cartridge 102 is, fortunately, unused. In those or other examples, the user 112 is able to quickly and easily identify that the battery 108 and the electrode pads 110 are unused. For instance, the user 112 looks at the AED 104 and views a display presenting text stating that the battery 108 and the electrode pads 110 are unused. Alternatively or additionally, the AED 104, in some examples, includes a battery indicator and an electrode pad indicator that light up in a color, such as green, and in a pattern, such as with a steady unblinking pattern, to show the user 112 that the battery 108 and the electrode pads 110 are unused. The AED 104, for instance, also indicates a level of charge of the battery 108 as being full, due to the battery 108 being unused.

In some examples, the AED 104 automatically turns on or activates the display/indicators when the user 112 picks up or sets down the AED 104. In those or other examples, the AED 104 turns on the display/indicators when the user 112 turns on the AED 104, such as by pressing a power button.

In various examples, the AED 104 automatically transmits a signal to an external device 120, such as a cell phone of the user 112. For instance, the AED 104 automatically transmits the signal to the cell phone based on the user 112 placing the cell phone next to the AED 104 to read a device, such as an NFC transceiver of the AED 104. The AED 104 and the external device 120 exchange data in some examples, based on the external device 120 being moved and based on a distance between the external device 120 and the AED 104 being less than a threshold distance. The signal, in some examples, indicates the battery 108 and the electrode pads 110 are unused, and possibly the charge level of the battery 108.

The user 112 is able to use any available options for identifying the AED 104 is new and ready, for example. In some cases, the user 112 does not try to obtain indication with the cell phone since the display/indicators of the AED 104 operate as assurance of the readiness of the AED 104.

In some cases, the user 112 opens the cartridge 102, which is removably coupled to the AED 104, and extracts and/or extends the electrode pads 110. The electrode pads 110, for example, include respective wires with respective ends connected to the cartridge 102. In some cases, the user 112 removes any clothing and/or objects covering areas of the patient 106 to which the electrode pads 110 are to be placed.

The user 112, for instance, attaches the electrode pads 110 to the skin of the abdominal and chest regions of the patient 106. The electrode pads 110 are attached, in some examples, based on the user 112 removing covers (e.g., plastic covers) over adhesive on the electrode pads 110. The user 112, for instance, places the electrode pads 110 on the patient 106.

In various cases, the AED 104 uses the electrode pads 110 to detect physiological conditions of the patient 106. For example, the AED 104 detects an electrical signal indicative of the ECG of the patient 106 via the electrode pads 110, and further detects that the ECG is indicative of VF by analyzing the ECG. The AED 104, in some cases, uses the VF detection to present text via the display, or to output audible commands via the speaker, of the AED 104. In various instances, presented text/audible commands are utilized by the AED 104 to recommend administration of a defibrillation treatment. For instances, the user 112, having quickly and efficiently, performed the various operations based on assurances by the AED 104 that the cartridge 102 is ready to be used, is able to identify that the defibrillation treatment, such as electrical shocks, are needed without delay.

In various cases, the AED 104, immediately and without delay after identifying that the defibrillation shocks are needed, charges a capacitor with electrical energy stored in the battery 108. The AED 104 also, for example and once the capacitor is charged, presents an indicator on the display representing the AED 104 being ready to be used for outputting the defibrillation shock. In some instances, the AED 104 presents a large indicator (or "ready indicator") associated with the AED 104 being ready. The user 112, for example, presses the indicator on the display, being a touchscreen. The AED 104, in some instances, transfers the electrical energy from the capacitor to the patient 106, via the electrode pads 110. As a result of the user 112 obtaining the information indicating that the cartridge 102 is new, the user 112 being able to act quickly to control the AED 104 to output the defibrillation shocks to the patient 106, the user 112 saves the life of the patient 106. The user 112, for example, performs complex treatment to save the patient 106, due to the user 112 being able to easily confirm the cartridge 102 is ready.

Although the subset(s) of the cartridge data 116 being initially stored in the cartridge 102 may be utilized by the cartridge 102, as discussed above in the current disclosure, it is not limited thereto. In some examples, any of the subset(s) of the cartridge data being initially stored in the cartridge 102 is stored during various processes, which are performed prior to any user related processes being performed. For instance, individual ones of the subset(s) of the cartridge data being initially stored are stored during manufacturing, configuration, quality service review, production, shipping, storing, stocking, inventorying, operating, various other processes of various types, or any combination thereof.

In some implementations, the cartridge 102 can be utilized by different types of medical devices. For example, the cartridge 104 may, in some cases, be configured to be coupled to the AED 104 and another type of external defibrillator, such as a monitor-defibrillator. In some implementations, the monitor-defibrillator utilizes the cartridge 102 differently than the AED 104. For example, in examples in which the cartridge data 116 indicates a manufacturing date of the cartridge 102, the AED 104 may determine that the cartridge 102 is unexpired as long as the manufacturing date is after a predetermined date associated with the AED 104. In contrast, the monitor-defibrillator, evaluating the cartridge 102 at the same time as the AED 104, may determine that the cartridge 102 is expired for use by the monitor-defibrillator because the manufacturing date is before a predetermined date associated with the monitor-defibrillator. That is, the predetermined date associated with the monitor-defibrillator may be after the predetermined date associated with the AED 104. These different predetermined dates may reflect slightly different uses of the cartridge 102 by the monitor-defibrillator with respect to the AED 104. For instance, the monitor-defibrillator may utilize a higher energy level for administering an electrical shock to a patient, as compared to the AED 104, such that the monitor-defibrillator may have higher quality expectations for the state of the electrode pads 110 before use.

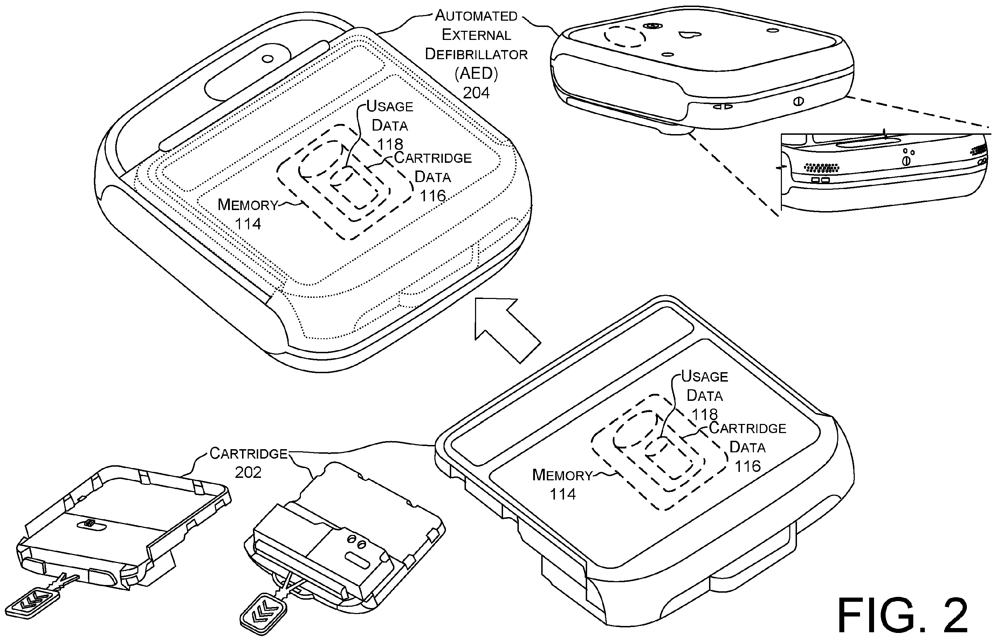

In some cases, the electrode pads 110 can be utilized multiple times before the cartridge 102 is considered, by the AED 104, to be expired. For instance, the electrode pads 110 may be utilized three times, on three separate patients, prior to the AED 104 determining that the cartridge 102 is expired. An indication of the multiple instances of use of the electrode pads 110, for instance, can be stored in the usage data 118. FIG. 2 illustrates example cartridges 202 and automated external defibrillators (AEDs) 204, the cartridges 202 configured to be removably coupled to the AEDs 204, the cartridges 202 including memory 206 utilized to store usage data 118. For example, any of the cartridges 202, any of the AEDs 204, and the memory 206 is utilized to implement the cartridge 202, the AED 104, and the memory 114, respectively, described above with reference to FIG. 1.

In some examples, a cartridge 202 is insertable in an AED 204. For instance, the cartridge 202 includes memory 206, which stores the cartridge data 116, including the usage data 118, as discussed above with reference to FIG. 1. In some cases, the cartridge 202 includes the electrode pads 110 and the battery 108, as discussed above with reference to FIG. 1. In some examples, the memory 206 includes one or more of various types of storage devices, such as a memory card, read only memory (ROM), flash memory, a micro secure digital (SD) card, a SD card, a compact flash card, programmable read-only memory (PROM), electrically erasable, programmable, read-only memory (EEPROM), a smart card, a subscriber identity module (SIM) card, or a radio frequency identification (RFID) chip.

In some cases, the cartridge 202 is inserted and connected in a cavity of the AED 204. For instance, the cavity includes one or more connectors (e.g., electrical connectors), and is configured to receive the cartridge 202. In some cases, the AED 204 includes one or more processors and memory.

In various implementations, the cartridge 202 and/or the AED 204 include one or more fasteners to secure the cartridge 202 to the AED 204. For example, the cartridge 202 and/or the AED 204 include one or more clips, one or more clasps, one or more straps, one or more lips, one or more hinges, one or more fasteners of any types, or any combination thereof.

In various examples, the AED 204 includes the cavity that has a sensor configured to sense that a potential at a port of the cavity is modified in response to coupling the port with a pin of a connector of the cartridge 202. For instances, the AED 204 analyzes identifier data (e.g., a signature of the cartridge 202), based on the AED 204 detecting the cartridge 202 is inserted, or is being inserted, in the cavity. In some examples, the AED 204 detecting the cartridge 202 includes the medical devices identifying the potential at the port of the cavity is modified, based on one or more signals being passed through one or more sensors in the port, and/or through one or more wires and/or conductors (e.g., one or more electrical conductors) with one or more corresponding ends connected to one or more corresponding terminals in the port.

In some cases, the AED 204 analyzes the signals to identify cartridge data (e.g., the cartridge data, as discussed above with reference to FIG. 1). For example, the AED 204 identifies a signature of the cartridge 202 in response to the sensing that the potential is modified.

In some examples, signal(s) received by the AED 204 include various types of data associated with the cartridge 202. For instance, the AED 204 receives the signal(s) and identifies data in the signal(s), including an identifier (e.g., the signature) and a status associated with the cartridge 202. In some cases, the identifier is unique to the cartridge, and the status indicates that the cartridge 202 is unused.

In various cases, the AED 204 stores various data associated with the cartridge 202 and performs various input/output. In some examples, the AED 204 receives from the cartridge 202, a value (or "usage value") (e.g., any of the values, as discussed above with reference to FIG. 1) in the cartridge data 116 and stores the value. In some cases, the cartridge 202 receives any of one or more subsets of the usage data 118, including the value and/or one or more other subsets of the usage data 118.

For instance, the usage value being received from the cartridge 202 and stored in the AED 204 symbolizes that the cartridge 202 is electrically coupled to the AED 204 and unused. In some examples, the AED 204 includes a display configured illuminate an indicator in response to the storing of the value. For instance, the display is operated to activate a mode of the indicator representing that the cartridge 202 is not used (e.g., activating the steady mode of the indicator representing that a cartridge is unused). In some examples, the display is operated to activate a steady mode of the indicator. The indicator, for example, is illuminated with a type of light (e.g., a steady light), and/or an audible alarm is not output (e.g., silenced).

In various implementations, such as with a cartridge being removably inserted in an AED and being used, the display is operated to activate a mode (e.g., a blinking mode) of the indicator representing that the cartridge is used (e.g., deactivate the steady mode, and activating the blinking mode of the indicator representing that the cartridge is used). The indicator, for examples, is illuminated with a type of light (e.g., a blinking light), and/or an audible alarm is output.

In some implementations, any one or more portions of the AED 204 are configured to receive any of the one or more portions of the cartridge 202. For example, the AED 204 includes one or more surfaces being open (e.g., sunk, inset, etc.) for receiving the cartridge 202. In some instances, one or more portions of either or both of two surfaces (e.g., a front surface and/or end, and a top (e.g., upward facing) surface and/or end) being perpendicular to one another, are open (e.g., sunk, inset, etc.) for receiving the cartridge 202.

Although any number of the portion(s) and/or the surface(s) of the AED 204 may be configured to receive any number of the portion(s) and/or the surface(s) of the cartridge 202, as discussed above in the current disclosure, it is not limited as such. In some examples, two sides of the AED 204 are open (e.g., sunk, inset, etc.) and four sides of the cartridge 202 fit against four sides of the AED 204, after the cartridge 202 is securely inserted into the AED 204. For instance, the cartridge 202 slides into the AED 204, with three sides and a top (e.g., upward facing surface, during operation) of the cartridge 202 contacting three internal sides and a sunken bottom (e.g., lower facing surface, during operation) of the AED 204.

In alternative examples, a single side of the AED 204 is open (e.g., sunk, inset, etc.) and five sides of the cartridge 202 fit against five sides of the AED 204, after the cartridge 202 is securely inserted into the AED 204. For instance, the cartridge 202 slides into the AED 204, with four sides and a top (e.g., upward facing surface, during operation) of the cartridge 202 contacting four internal sides and a sunken bottom (e.g., lower facing surface, during operation) of the AED 204.

In various cases, the usage data 118 includes various types of data representing various aspects related to usage, including previous usage, of a portion (e.g., a partial portion or an entire portion) the cartridge 202. One or more portions of the cartridge 202 include, for example, the component(s) (e.g., the sub-devices), circuits, any other electrical and/or electronic portions of the cartridge 202, or any combination thereof.

In some examples, the usage data 118 is utilized to represent individual ones of usages of corresponding sub-devices of the cartridge 202. In some examples, the sub-device(s) include the electrode pads 110, the power supply devices (e.g., the battery 108). In those or other examples, the sub-device(s) include the processor(s) (e.g., the ASIC), the sensor(s), the visual indicator device(s), the converter(s), the speaker(s), the microphone(s), the Ul(s), the mechanism(s), various types of other sub-devices, or any combination thereof.

In various cases, the cartridge data 116 is utilized by the user 112 to verify one or more statuses (e.g., the status data, as discussed above with reference to FIG. 1) of the cartridge 202. For instance, the status data is included in the cartridge data 116. In some examples, the cartridge data 116 is utilized to verify whether the cartridge 202 is in operating condition. In those or other examples, the cartridge data 116 is utilized to verify whether any portion of the cartridge 202 is fully functional, unused, authentic, sanitary, structurally sound, satisfactory in any other way, or any combination thereof.

In various implementations, the cartridge data 116 includes data which may be crucial for safe, predictable, and effective operation of the cartridge 202. For instance, the cartridge data 116 enables the cartridge 202 to be confidently and reliably utilized along with the AED 204. In contrast to existing AEDs, including AEDs without removable cartridges, AEDs without cartridge data, and AEDs without usage data, the AEDs being implemented according to the techniques discussed herein, such as the AED 204, for example, are utilized to manage significant and important data. In some cases, the cartridge 202 provides (e.g., visibly and/or audibly presents) output representing the cartridge data 116 to the user 112 as the user 112 begins using the AED 204.

In some implementations, the AED 204 processes one or more requests (e.g., identifier requests) for one or more identifiers. For instance, the request(s) are utilized by the user 112 or one or more other users to obtain the identifier(s). For example, the request(s), are processed by the AED 204 based on one or more communications from the external device(s) 120. In various cases, the request(s) are routed to, and processed by, the cartridge 202. In some instances, the request(s) are processes based on one or more selections received via user input from the user(s) 112. For example, user input utilized to control the AED 204 to process the request(s) includes touch input to one or more single indicators, tactile input to one or more usage buttons, audio input of one or more simple commands (e.g., a voice command of "is the cartridge used"), and/or any other type of user input.

In some implementation, the identifier request(s) include one or more inquiries that are associated with corresponding combinations of components of the cartridge 202 and/or software subsets of the cartridge 202. Alternatively or additionally, the identifier request(s) include individual ones of one or more inquiries corresponding to the cartridge 202 itself (e.g., all of the component(s) of the cartridge 202, and/or all of the software subset(s) of the cartridge 202), in its entirety (e.g., as a whole).

In various cases, the identifier request(s) include one or more usage data requests. In some instances, the usage data request(s) are utilized to retrieve the usage data 118. In those or other examples, the usage data requests (or "usage data inquiry(ies)") are associated with one or more corresponding components of the cartridge 202 and/or one or more corresponding software subsets of the cartridge 202.

In various cases, the usage data request(s) are not required to include any specific types of request data, such as one or more names, one or more numbers, one or more passwords, etc. associated with the user 112, the user(s), and/or their device(s). For instance, the usage data request(s) are not required to identify the user 112 controlling the external device 120. By not requiring the usage data request(s) to include identifier data, for example, simplicity and reliability of operations being performed based on the usage data request(s) are ensured. In some cases, the identifier request(s) associated with only the cartridge 202 (e.g., not with any specific sub-devices of the cartridge 202) are not required to include the identifier data. Alternatively or additionally, the identifier request(s) associated with corresponding sub-devices of the cartridge 202 are not required to include the identifier data.

In some examples, the identifier request(s) associated with other data than the usage data 118 are required to include specific types of request data. For instance, individual ones of the identifier request(s) utilized to receive important data, security related data, certain types of the component data, etc., are required to include the specific types of request data. For such an instance, the identifier request(s) are required to include the specific types of request data before the AED 204 proceeds with operations processed based on the identifier request(s). The specific types of request data include, for example, the name(s), the number(s), the password(s), etc. associated with the user 112, the user(s), and/or their device(s).

In various cases, one or more identifier responses, such as one or more usage data responses, are output (e.g., transmitted and/or visually/audibly output) based on the identifier request(s) in various ways. For example, the usage data response(s) include one or more results generated by the AED 204, based on the identifier request(s). In various cases, result(s) include one or more identifier results (or "usage data result(s). In some cases, the identifier result(s) are output, such as via one or more communications, and/or via one or more illuminations of one or more corresponding usage indicators. In some examples, the identifier result(s) are output by the AED 204 presenting the identifier result(s) via the display.

In some examples, the identifier response(s), such as the usage data response(s), are managed in a similar way as for the notification(s). For instance, the identifier response(s) are generated based on the cartridge data 116. In some cases, the identifier response(s) are generated based on any other data stored in the cartridge 202 and/or AED 204. For instance, such as with an identifier request being received by the AED 204 to verify whether the cartridge 202 is new, generating a usage data response includes obtaining the usage value from the usage data 118 and providing it as output via the usage data response(s).

In such an instance or another instance, with examples in which an identifier request is utilized to verify whether the cartridge 202 is new, generating a usage data response includes comparing the signature in the cartridge data 116 with the cartridge signature in the data stored in the AED 204. For instance, the AED 204 then identifies the usage value stored the cartridge 202, based on the cartridge signature in the cartridge data 116 matching the cartridge signature stored in the AED 204. In such an instance or another instance, the usage data response is output to indicate, based on the usage value (e.g., based on the usage value having a value of "0") that the cartridge 202 is usable. For example, an indicator (or "new cartridge indicator") associated with the cartridge 202 being new is illuminated, the display of the AED 204 presents the numeral "0" as the usage indicator, the display of the AED 204 switches from illuminating any of one or more other portions (e.g., one or more other indicators) of the display to illuminating the usage identifier, the cartridge 202 and/or the AED 204 perform any of various other operations indicating the cartridge 202 is new, or any combination thereof.

In various cases, the AED 204 possibly requires one or more communications to be received in response to the identifier response(s) before continuing with any operations. In various cases, the AED 204 possibly requires the communication(s) to be received based on the identifier request(s) being associated with the battery 108 and/or the electrode pads 110. In various cases, the AED 204 possibly requires the communication(s) to be received based on the identifier request(s) being utilized to confirm any compatibility and/or functionality of the cartridge 202 and/or with the relatively more important sub-device(s). For instance, the AED 204 possibly requires the communication(s) to be received based on the identifier request(s) being utilized to confirm compatibility and/or functionality of the battery 108 and/or the electrode pads 110. In some cases, the confirmation(s) are received based on the user 112 observing and confirming that the output is correct. For instance, the user 112 is able to confirm that the identify results data being output is correct.

For example, the confirmation(s) are received via the communication(s) being exchanged and/or user input being received. In various cases, the communication(s), and/or the selection(s) via user input, are received and utilized by the AED 204. For instance, the communication(s), and/or the selection(s), are received and utilized to identify whether the AED 204 is to proceed or to halt. In some cases, the AED 204 proceeds or halts individual ones of one or more operations of the cartridge 202 and/or the AED 204. For instance, the AED 204 enables the operation(s) to proceed in response to outputting the confirm notification(s) and/or the confirm response(s). In such an instance, the AED 204 enables the operation(s) to proceed in response to receiving the confirmation(s).

In some cases, the confirmation(s) include acknowledgements (e.g., one or more positive acknowledgements). For example, the acknowledgements indicate that the data in the notification(s) and/or the identifier response(s) is correct, satisfactory, includes values within thresholds, indicates that the portion(s) of the AED 204 are compatible, or any combination thereof, the AED 204 proceed with various operations. For example, based on the confirmation(s), the cartridge 202 and/or the AED 204 proceed with any normal operation(s) and/or allow the user(s) to control the cartridge 202 and/or the AED 204 to proceed with the operation(s).

Although the AED 204 may halt various operations until the confirmation(s) are received, as discussed above in the current disclosure, it is not limited as such. In some examples, the AED 204 proceeds with various operations without waiting for the confirmation(s). In some cases, the settings data is manageable to enable the AED 204 to stop and wait for the confirmation(s). In some cases, the settings data is manageable to enable the AED 204 to automatically proceed with the operation(s) without requiring confirmation(s) to be received.

For example, the settings data is manageable to enable the AED 204 to operate differently with respect to confirmation(s) associated with different types of notifications and/or responses. In some cases, the settings data is usable for overriding any default halts of operations associated with some types of notification(s) and/or response(s). The settings data is usable, for instance, to ensure that the AED 204 is usable for any operations(s) even if the usage data notification(s) are output. The settings data is usable, for instance, to ensure that the AED 204 is usable for any operations(s) even if the usage data notification(s) indicate that the cartridge is used.

In other examples, the AED 204 is controlled in various ways based on the communication(s) and/or the selection(s) including one or more negative acknowledgements. For example, the negative acknowledgment(s) may indicate that the user 112 identifies output data in the notification(s) and/or the response(s) as being incorrect or unsatisfactory. In some cases, the negative acknowledgment(s) may indicate that the user 112 identifies usage values in the output data exceeding thresholds (e.g., a usage value being greater than 0). For example, the values may be associated with the portion(s) of the cartridge 202 and/or the portion(s) of the AED 204 being incompatible, unsatisfactory, or any combination thereof.

In some examples, the AED 204 performs various operations based on the negative acknowledgement(s). For instance, the AED 204 disables individual ones of the portion(s) of the cartridge 202 and/or individual ones of the portion(s) of the AED 204. In those or other examples, the AED 204 deactivates various portion(s) of the cartridge 202 and/or the AED 204. In those or other examples, the AED 204 powers-down the cartridge 202 and/or the AED 204.

In some cases, incompatibility associated with component data may be indicated in the output data. For instance, the output data may include a current identifier that is incorrect. In such an instance, the identifier may include a serial number for a part that is different from an initial serial number (e.g., a valid serial number). In some cases, the output data may include an identifier associated with manufacturing, shipping, authorization, etc., of the cartridge 202.

In some instances, to increase efficiency, safety, and reliability of the AED 204, the negative acknowledgement(s) are utilized to automatically disable or power down various portions of the cartridge 202 and/or the AED 204. In such instances, the power downs are utilized to provide optimal power management for increasing the life of the battery 108. In such instances, individual ones of the sub-device(s) of the cartridge 202 and/or the AED 204 are possibly disabled, disconnected from any other sub-devices and/or circuits, etc. In some instances, the power down or disabling is performed by a switch being opened between the battery and one or more other sub-devices being opened. In some instances, the power down or disabling is performed to increase efficiency, safety, and reliability of the cartridge 202 and/or the AED 204.

In some cases, controlling the AED 204 to proceed or not proceed with the operation(s) based on negative acknowledgement(s) being received is based on the settings data. In some instances, the AED 204 uses the settings data to determine to disable components, or to halt or discontinue operations, automatically. For example, the settings data controls the AED 204 to disable components, or halt operations, in response to receiving negative acknowledgement(s) for some types of notifications and/or responses. In some cases, disabling components or halting operations ensures that the user 112 is aware that the identifier notification(s) and/or the identifier response(s) were output. In some cases, disabling components or halting operations ensures that the user 112 is aware that the battery 108 and/or the electrode pads 110 are used. In some instances, the AED 204 uses the settings data to determine to perform operations automatically notwithstanding the negative acknowledgement(s) being received.

In various examples, the settings data is utilized by the AED 204 to determine how to operate after the identifier notification(s) and/or the identifier response(s) are output. For instance, the AED 204 automatically discontinues operations, and/or controls various portion(s) of the cartridge 202 and/or the AED 204 to be automatically disabled, in response to outputting the identifier notification(s) and/or the identifier response(s). In various instances, operations halt, and/or the portion(s) are automatically disabled, based on the identifier notification(s) and/or the identifier response(s) indicating electrode pads or a battery are not new. In such an example, controlling the AED 204 to discontinue operation(s) and/or to disable various portion(s) of the cartridge 202 and/or the AED 204 allows the user(s) to correct any problems. In such an example, discontinuing operation(s) and/or to disabling various portion(s) of the cartridge 202 and/or the AED 204 allows the user(s) to replace used electrode pads with new electrode pads or replace the used battery with a new battery.

Although communication(s) and/or the selection(s) (or "user selection(s)") may be received in various ways, as discussed above in the current disclosure, it is not limited thereto. For instance, any type of communication(s) and/or any type of user input (e.g., user input including touch input, audio input, mechanical input, any other type of input, or any combination thereof), is received in a similar way as any of the communication(s) and/or the user input for purposes of implementing any of the techniques discussed herein. In various cases, one or more subsets of data identified by any of the selection(s) are received by touch input to a touchscreen. For example, touch input may be to one or more identifiers (e.g., the name(s), the number(s), etc.), one or more symbols (e.g., bullets, names (e.g., part names), numbers (e.g., part numbers), consecutive numbers and/or letters in one or more lists, one or more tables, one or more graphs, one or more charts, etc. In some instances, the touch input may be in response to any other types of symbols being presented by the touchscreen.

In various examples, the user selection(s) are received via one or more audible commands. For example, the user selection(s) are received via one or more voice commands, one or more audible utterances, etc., or any combination thereof. In some cases, the user selection(s) are received via audible input to a microphone. For example, the audible input is utilized by the AED 204 to capture and/or generate digital data (e.g., digital audio data) identifying any of the identifier data (e.g., any of the name(s), any of the number(s), and/or any other symbol of any type representing the name(s), any of the number(s), or any combination thereof).

Although the identifier request(s) may be received in various ways, as discussed above in the current disclosure, it is not limited as such. In various examples, the identifier request(s) being exchanged via the communication(s) are processed as one or more requests (or "ping(s)") for any types of identifier data and/or any other type of data. In various examples, the identifier request(s) are processed as the request(s) for characteristics data. The characteristics data includes, for instance, any of the characteristic(s) discussed herein.

In some cases, the identifier request(s) are utilized to obtain individual ones of the identifier(s). For example, the identifier request(s) are utilized to obtain the name(s), the date(s), etc., as discussed above in FIG. 1. In some instance, the identifier request(s) are utilized to obtain a corresponding product name, a corresponding manufacturer name, etc., or any combination thereof. For example, the name(s) are associated with corresponding sub-devices, software, etc., or any combination thereof. In some instances, the identifier request(s) are utilized by the AED 204 to identify, and to output (e.g., transmit and/or audibly/visually output), the name(s) and/or the date(s). In some instances, the AED 204 outputs response data, including the name(s) and/or the date(s), in the corresponding identifier response(s).

In various examples, the identifier request(s) include various types of request data. For instance, the request data includes a corresponding serial number, a corresponding part number, etc. or any combination thereof associated with corresponding sub-devices, software, etc., or any combination thereof. In some cases, the identifier request(s) are utilized by the AED 204 to identify, and to output, any other types of corresponding identifiers. For example, the identifier(s) are included in the response data being output in the corresponding identifier response(s), based on the request data. The response data includes, in some cases, corresponding names, such as a corresponding product name, a corresponding manufacturer name, etc., or any combination thereof. The response data includes, in some cases, corresponding date(s).

In some examples, the identifier response(s), which include the response data, are output in various ways. For instance, the identifier response(s) are output (e.g., transmitted or presented) visually by at least one display of the AED 204. In various cases, the identifier response(s) are audibly output by at least one speaker of the AED 204. In some examples, the identifier response(s) are emitted as illumination of one or more indicators of the AED 204. In some examples, the identifier response(s) are output any other way by the AED 204, such as by any combination of any of the types of output. In various cases, the identifier response(s) are output as a list, such as an itemized list of the number(s), the name(s), etc. For instance, the itemized list includes the number(s), the name(s), etc., associated with corresponding ones of the sub-devices and/or corresponding ones of the software subsets. The list, in some cases, is presented by the display, output any other way, or any combination thereof, based on the identifier response data.

In various implementations, the AED 204 includes one or more indicators associated with usage of the cartridge 202 and/or one or more sub-devices having relatively higher importance levels. In some examples, any of the indicator(s) are associated with usage of the cartridge 202, usage of the electrode pads 110, usage of the battery 108, usage of other sub-devices of the cartridge 202, or any combination thereof. In some cases, the indicator(s) are controlled to have steady illumination to indicate that the corresponding sub-devices are new, and/or the indicator(s) are controlled to blink to indicate the corresponding sub-devices are used.

Although settings data may be managed by the AED 204, as discussed in the current disclosure, it is not limited thereto. In some examples, the settings data, as briefly discussed above with reference to FIG. 1, is utilized to generate, modify, and/or manage the cartridge data 116 in various ways. In some examples, the settings data is initially stored in the AED 204. In those or other examples, the settings data is generated and/or modified by one or more communications. In those or other examples, the settings data is generated and/or modified based on user input.

The settings data, for instance, is utilized to indicate whether any of the identifier request(s) are required, or not required, to include the identifier data. In some cases, the settings data is utilized to control the AED 204 to not require identifier data for the usage data request(s). In various cases, the settings data is utilized to control the AED 204 to not require identifier data for operations associated with convenience or aesthetics. In some instances, the settings data is utilized to control the AED 204 to require the identifier data for individual ones of the identifier request(s) that are related to relatively more important sub-devices (e.g., the processor(s), the transceiver(s), etc.).

In various cases, the settings data is managed via the communication(s) and/or the user input based on the cartridge 202, and/or the sub-device(s) therein, not being new. For instance, the settings data is utilized to control the AED 204 to perform operation(s) and/or to halt based on the cartridge 202, and/or the sub-device(s) therein, not being new. For example, the settings data is utilized to control the AED 204 to perform normal operations notwithstanding the usage data 118 indicating that based on the cartridge 202, and/or the sub-device(s) therein, are not new. In such an example, the settings data is utilized to control the AED 204 to enable treatment to be provided to the patient 106, such as treatment that is crucial and/or urgent.. In such an example, the settings data is utilized to enable the AED 204 to be used in such occurrences where another cartridge is unavailable or not easily obtainable. In those or other examples, any restriction(s) may be overridden and/or ignored based on the settings data.

In some implementations, one or more values (e.g., associated with one or more selections) of the setting(s) are received via user input. In various examples, the value(s), such as one or more updates are received based on one or more communications and/or one or more selections provided via user input. In those or other examples, the value(s) are received based on one or more current settings being output (e.g., transmitted, or audibly/visually output by the AED 204). For instance, the current setting(s) are output by the AED 204 presenting the current setting(s) via the display. In those or other examples, the setting(s) are communicated, output visually via presentation of the setting(s) by one or more displays of the AED 204, output audibly by one or more speakers of the AED 204, and/or output via one or more other types of output in similar ways.

In various examples, the setting(s) identify whether output of various identifier notification(s) and/or identifier response(s) are automated and/or activated, or not. In some cases, the setting(s) identify whether the AED 204 is to automatically proceed with, and/or to automatically halt, any operation(s). For instance, the setting(s) identify whether the AED 204 is to automatically proceed or halt, prior to receiving confirmation(s).

Although the AED 204 may include various input/output components and/or performs input/output functions, as discussed above in the current disclosure, it is not limited as such. In various cases, such as with input being received and/or data being output, the cartridge 202 incudes one or more of any types of components (e.g., input/output components and/or external communication components) being similar to any of the components (e.g., the input/output components and/or external communication components) of the AED 204, for purposes of implementing any of the techniques discussed herein.

In those or other cases, the cartridge 202, the AED 204, or any combination thereof, performs any functions similar to any of the functions performed by the AED 204, for purposes of implementing any of the techniques discussed herein. For example, the cartridge 202, the AED 204, or any combination thereof, performs any input/out and/or external communications, in a similar way as input/output and/or external communications performed by the cartridge 202 and/or the AED 204. In some examples, by utilizing the cartridge 202, the AED 204, or any combination thereof to perform any types of functions, the cartridge 202, the AED 204, or any combination thereof, may be customized in any way. In some examples, the cartridge 202, the AED 204, or any combination thereof may be customized such as to provide various options for the user 112 and/or the external device(s) 120 to interact with the cartridge 202, the AED 204, or any combination thereof. In those or other examples, the user 112 and/or the external device(s) 120 interact with the cartridge 202, the AED 204, or any combination thereof to provide various options for external communications to be exchanged with the cartridge 202 and/or the AED 204).

In some implementations, input/output components and/or communication components are omitted from the cartridge 202. For instance, components utilized to communicate with the external device(s) 120 are omitted from the cartridge 202. By not including the input/output components and/or communication components in the cartridge 202, the cartridge 202 may be simplified to reduce a likelihood of a malfunction occurring in the cartridge 202. Simplifying the cartridge 202 enables costs and/or requirements associated with manufacturing, production, maintenance, distribution, operation, and/or any other aspects of the cartridge 202, to be reduced and/or minimized.

For instance, the cartridge 202 may be designed, assembled, manufactured, etc., to be extremely simplified, such as to include on minimal components needed for the AED 204 to operate efficiently and reliably. In some examples, the cartridge 202 may include the memory 114, the electrode pads 110, the battery 108, the processor(s), the circuit(s), and any other required sub-devices. In those or other examples, the cartridge 202 may not include any optional sub-devices. For instance, the cartridge 202 may not include any input/output devices, and/or any complex electrical components, such as the ADC(s), and/or any other types of the component(s) in the AED 204.

By omitting various components in the cartridge 202 that are unnecessary for operation of the AED 204, the cartridge 202 may be configured to be disposable (e.g., environmentally, cost effectively, etc., disposable). The cartridge 202 may be usable with a high degree of reliability, since the cartridge 202 includes fewer components that might break and/or cause operability problems, thereby enabling the user 112 to confidently and successfully medically treat, and in some cases, to save the life of, the patient 106.

Although the usage data 118 may be managed in various ways as discussed above in the current disclosure, it is not limited as such. In some cases, the usage data 118 is updated based on one or more types, and/or one or more characteristics, of the electrode pads 110, and/or any changes thereto. For instance, the usage data 118 is updated based on the AED 204 identifying any changes to the characteristic(s). The AED 204, in various cases, identifies the type(s) and/or the characteristic(s) based on the identifier(s) and/or any of the sensed data. In some examples, the cartridge data 116, such as the component data, indicates the type(s) of electrode pads. In some cases, the characteristics data in the component data include the type(s) and/or the characteristic(s) associated with the electrode pads 110. In those or other examples, the usage data 118 is itemized, linked to, and/or managed based on, any of the type(s) and/or the characteristic(s). For instance, the usage data 118 is updated based on any modifications to any of the type(s) and/or the characteristic(s) of the electrode pads 110.

In various implementations, the cartridge data 116 includes one or more identifiers of the type(s) and/or the characteristic(s) of the electrode pads 110. In some instances, the cartridge data 116 includes the identifier(s) associated with any aspects and/or characteristics of electrode pads 110 and/or any sub-device(s) of the cartridge 202.

Various types of electrode pads may be utilized with the cartridge 202. For example, the type(s) of electrode pads include, disposable electrode pads, recyclable electrodes pads, or any combination thereof. In some cases, the electrode pads 110 include electrode pads (or "normal electrode pads") used for normal operations of the cartridge 202 and the AED 204, electrode pads (or "training electrode pads") used for training operations, or any combination thereof.

In some examples, the normal electrode pads are uniquely suited to operations being performed during emergency scenarios. In some instances, the training electrode pads are uniquely suited to operations not being performed during emergency scenarios.

The characteristic(s) of the normal electrodes pads include, for example, the fully-adhesive gel, such as for single usage. The fully-adhesive gel enables secure and strong connections between the normal electrodes pads and the patient. The characteristic(s) of the training electrodes pads include, for example, the semi-adhesive gel for repeat usage.

In some cases, the characteristic(s) of the normal electrode pads include metal and/or plastic of a quality with a level that is above a threshold level of quality, and/or above a level of quality of metal and/or plastic of the training electrode pads. In some cases, the characteristic(s) of the normal electrode pads include circuits of a quality with a level that is above a threshold level of quality, and/or above a level of quality of circuits of the training electrode pads.

In some examples, the characteristic(s) of the normal electrode pads include a level of adhesiveness of that is larger than a threshold normal level, larger than the level of adhesiveness of the training electrode pads, or any combination thereof. In some examples, the characteristic(s) of the training electrode pads include a level of adhesiveness that is smaller than a threshold training level of adhesiveness (e.g., the same threshold level of adhesiveness as the threshold normal level of adhesiveness or a different threshold level of adhesiveness), smaller than the level of adhesiveness of the normal electrode pads, or any combination thereof.

The types of electrode pads include, for instance, adult electrode pads, child electrode pads, pediatric electrode pads, any other age-group oriented electrode pads, or any combination thereof. In some examples, the characteristic(s) of the adult electrode pads include an energy level (e.g., a level of energy being produced during energy delivery) that is larger than a threshold adult energy level, larger than the energy level of the child electrode pads, larger than the energy level of the pediatric electrode pads, or any combination thereof. In some examples, the characteristic(s) of the adult electrode pads include a size and/or weight that is larger than a threshold adult size and/or weight, larger than a size and/or weight of the child electrode pads, larger than a size and/or weight of the pediatric electrode pads, or any combination thereof.

In some examples, the characteristic(s) of the child electrode pads include an energy level that is larger than a threshold minimum child energy level, smaller than the energy level of a threshold maximum child energy level, smaller than the energy level of the adult electrode pads, larger than the energy level of the pediatric electrode pads, or any combination thereof. For instance, the characteristic(s) of the child electrode pads include a smaller threshold energy level than the threshold adult energy level, and/or a smaller threshold energy level than the threshold maximum child energy level. In some examples, the characteristic(s) of the child electrode pads include a size and/or weight that is larger than a threshold minimum child size and/or weight, smaller than a threshold maximum child size and/or weight, smaller than the size and/or weight of the adult electrode pads, larger than the size and/or weight of the pediatric electrode pads, or any combination thereof. For instance, the characteristic(s) of the child electrode pads include a smaller threshold size and/or weight than the threshold adult size and/or weight, and/or a smaller threshold size and/or weight than the threshold maximum child size and/or weight.

In some examples, the characteristic(s) of the pediatric electrode pads include an energy level that is smaller than a threshold maximum pediatric energy level, larger than a threshold minimum pediatric energy level, smaller than the energy level of the adult electrode pads, smaller than the energy level of the child electrode pads, or any combination thereof. In some examples, the characteristic(s) of the pediatric electrode pads include an energy level that is a smaller threshold energy level than the threshold minimum child energy level. In some examples, the characteristic(s) of the pediatric electrode pads include an energy level that is a smaller threshold size and/or weight than the threshold maximum pediatric energy level. In some examples, the characteristic(s) of the pediatric electrode pads include a size and/or weight that is smaller than a threshold maximum pediatric size and/or weight, larger than a threshold minimum pediatric size and/or weight, smaller than the size and/or weight of the adult electrode pads, smaller than the size and/or weight of the child electrode pads, or any combination thereof. In some examples, the characteristic(s) of the pediatric electrode pads include a size and/or weight that is a smaller threshold size and/or weight than the threshold minimum child size and/or weight. In some examples, the characteristic(s) of the pediatric electrode pads include a size and/or weight that is a smaller threshold size and/or weight than the threshold maximum pediatric size and/or weight.

In some examples, various types of power supply devices are usable to supply power to the cartridge 202. For instance, types of power supply devices that are identifiable in the characteristics data include one or more batteries (e.g., the battery 108), one or more capacitors, one or more of any other types of power supply device, or any combination thereof.

Although the cartridge data 116 may be stored for the cartridge 202 and/or the sub-devices, including the electrode pads 110 and/or the battery 108, as discussed above in the current disclosure, it is not limited as such. In some examples, the cartridge data 116 includes various types of data. For instance, the cartridge data 116 includes numerical data, boolean data, binary data, any other type of data, or any combination thereof. For instance, with examples in which the usage data 118 includes numerical data, the numerical data includes a value representing a number of previous usages of the cartridge 202. In some examples, previous usages include occurrences of previous usages of the cartridge 202, previous incidents of electrical activity, previous activations of the cartridge 202, operations of the cartridge 202 being previously performed, or any combination thereof.

In some cases, the previous usages being stored in the usage data 118 include various types of usages. For instance, such as with values representing previous usages being stored in the usage data 118, the previous usage(s) include corresponding incidents of electrical activity, corresponding activations, corresponding operations, any other corresponding types of previous usages, or any combination thereof.

In some cases, such as with examples in which the value representing the number of the previous incidents of electrical activity is stored in the usage data 118, the previous electrical activity incidents include power being supplied to sub-devices of the cartridge 202.

In some cases, such as with examples in which the value representing the number of the previous activations is stored in the usage data 118, the previous activations include previous power-ons of the cartridge 202. The previous power-ons include, for instance, successful power-ons of (e.g., successful power supply to) all of the sub-devices of the cartridge 202. In some examples, the previous power-ons include a power-on selection being received via user input, power being supplied to the sub-devices, initialization of the sub-devices, and automated power-on operations being performed by the sub-devices.

For instance, with examples in which the usage data 118 includes data indicating the occurrences of the previous usages of the cartridge 202, the data indicating the occurrences of the previous usages includes the value representing a number of occurrences of previous usages of the cartridge 202. For example, the number of occurrences represents cartridge usages, which includes previous usages (or "cartridge usages") of the cartridge 202. In some instances, any of the values (or "tic marks") included in the usage data 118 represent corresponding numbers of corresponding occurrences of previous usages of corresponding sub-devices of the cartridge 202.

By utilizing various usage types to track and/or provide outputs representing the cartridge usages, for example, amounts of memory resources of the cartridge 202 and/or the AED 204 are optimizable and/or adjustable. By utilizing the tic marks, for example, less amounts of data are required to be stored than by utilizing dates of usage, numbers of usage, etc., which decreases an amount of memory required to be included the cartridge 202.

In some cases, such as for cases in which the usage data 118 is utilized by the cartridge 202 to indicate that the cartridge 202 was not previously used, the usage data 118 is utilized by the cartridge 202 to indicate how many times the cartridge 202 was previously used. For example, the usage data 118 includes a value representing how many time the cartridge 202 was previous used, the value being zero, representing the cartridge 202 was previous used zero times.

Although the cartridge 202 may be updated in various ways based on usage(s) and/or change(s) in battery level(s), as discussed above in the current disclosure, it is not limited as such. For instance, the cartridge 202 is automatically updated, such as without requiring communication(s) and/or user input. In some cases, the cartridge 202 is automatically updated based on usage(s) and/or change(s) in battery level(s). In various examples, the cartridge 202 is utilized to manage data that is updated before, during, or after usage, such as by one or more users of various types. For example, the user(s) include any type of user, such as any of one or more operators, any of one or more technicians, any of one or more other types of users, or any combination thereof.

In those or other examples, the cartridge 202 is utilized to manage data that is updated before, during, or after usage, such as by one or more selections via user input to the AED 204 and by the user. For instance, in such case as is necessary, the user navigates one or more menus of the AED 204 and provides the selection(s) to adjust one or more values stored in the AED 204 associated with the electrode pads 110 to change the value(s) from new and/or unused to be used and/or not new. The selection(s), in some cases, are in response to the AED 204 outputting a notification to request the user to confirm the status of the AED 204 being new and/or unused, and the selection(s) being provided based on the notification to change the status to used and/or not new (e.g., such as if the user is aware of electrode pad(s) in the cartridge 202 being used and/or not new).

Although the technician/operator may usually have knowledge and familiarity with cartridges to update the cartridge data 116, as discussed above in the current disclosure, it is not limited as such. In various examples, the cartridge data 116 is updated, and/or any other function(s) associated with the AED 204, or any combination hereof, are performed by any of one or more types of users, including the user 112, any other user, or any combination thereof, in a similar way as for the technician/operator and/or the user 112 for purposes of implementing any of the techniques as discussed herein.

Although the user 112 may often be present at an occurrence of a medical emergency of a patient(s) (e.g., the patient 106) to operate the AED 204, as discussed above in the current disclosure, it is not limited as such. In various examples, the AED 204 is utilized and/or controlled by the technician/operator, the user 112, any other user(s), or any combination thereof, to provide any function(s) associated with the cartridge 202 and/or the AED 204, in a similar way as for the user 112 for purposes of implementing any of the techniques as discussed herein.

Although the cartridge 202 and/or the AED 204 may include the various component(s), operate utilizing the data of various types, and/or perform the various function(s), as discussed above in the current disclosure, it is not limited as such. In some examples, the cartridge 202 includes any of the various component(s), operates utilizing any of the data of various types, and/or performs any of the various function(s) of the AED 204, for purposes of implementing any of the techniques as discussed herein. In those or other examples, the AED 204 includes any of the various component(s), operates utilizing any of the data of various types, and/or any of performs the various function(s) of the cartridge 202, for purposes of implementing any of the techniques as discussed herein.

Although the AED 204 may be a type of medical device configured to be removably connected to the cartridge 202, as discussed above in the current disclosure, it is not limited as such. In various examples, one or more of any types of medical devices may be utilized in a similar way as for the AED 204 for purposes of implementing any of the techniques as discussed herein.

Although various types of data may be included in communications as discussed throughout the current disclosure, it is not limited as such. In some examples, any of the communication(s) include one or more wireless communications (e.g., one or more transmitted wireless communications and/or one or more received wireless communications) and/or one or more wired communications (e.g., one or more transmitted wired communications and/or one or more received wired communications) with any devices (e.g., any of the external device(s) of any types) for purposes of implementing any of the techniques discussed herein.

Although the user(s) of the cartridge 202 and/or the AED 204 may include various types of users, as discussed above in the current disclosure, it is not limited as such. For instance, the user(s) include the facility personnel, the maintenance personnel, the medical personnel, the other personnel of various types, or any combination thereof, as discussed above with reference to FIG. 1. In some examples, the user(s) include one or more users of the external device(s) 120, the user 112, the cartridge 202, and/or. In some examples, the user(s) may be interpreted as including the user 112, or vice versa, for purposes of implementing any of the techniques as discussed herein. In those or other examples, the external device(s) of the user(s) may be interpreted as including the external device(s) of the user 112, or vice versa, for purposes of implementing any of the techniques as discussed herein.

Although the identifier data may be utilized by the AED 204 in various ways, as discussed above in the current disclosure, it is not limited as such. In various examples, the identifier data includes one or more numbers (e.g., various types of numbers, including unique numbers) and/or names (e.g., various types of names, including unique names) associated with the cartridge 202 and/or the sub-devices of the cartridge 202. For instance, the identifier(s) include a signature (e.g., a product signature, a component signature, a device signature, etc., or any combination thereof), a serial number, a product identifier, an inventory identifier, a tracking identifier, a shipping identifier (e.g., a shipping order number, etc.), a manufacturer identifier, an internal identifier, any other identifiers of other types, or any combination thereof.

Although the identifier(s) may include various types of identifiers as discussed above in the current disclosure, it is not limited as such. In various examples, the identifier(s) include one or more name, one or more unique numbers, one or more part numbers, one or more internal identifiers, or any combination thereof. In some cases, the internal identifier(s) is utilized by the software of the cartridge 202 and/or the AED 204 to track any of the cartridge data 116. In some examples, the signature includes, for example, one or more ascii values, one or more numerical values, one or more alphabetical values, one or more binary values, one or more hexadecimal values, one or more values of any other types, or any combination thereof, as a unique code representing a device/sub-device (e.g., the cartridge 202).

In some examples, the identifier(s) include the date data, which includes one or more dates (e.g., various types of dates, including unique dates) associated with the cartridge 202 and/or the sub-devices of the cartridge 202. For instance, the date(s) include includes an expiration date, a manufacturing date, a shipping date, or any other dates of other types, or any combination thereof.

Although outputs of various types may be performed by the AED 204, as discussed above in the current disclosure, it is not limited as such. In some examples, the external device(s) 120 are utilized to request one or more updates to the AED 204. For instance, the update(s) may include or more cartridge updates to be utilized by the AED 204 to update the cartridge 202. In some cases, the update(s) are utilized by the AED 204 to output different amounts of information. For example, the update(s) are utilized to cause the AED 204 not currently outputting the usage data 118 to output the usage data 118.

Although communication(s) and/or user input may be utilized to control the AED 204 in various ways, as discussed above in the current disclosure, it is not limited as such. In various implementations, the communication(s), and/or the selection(s) via user input, are utilized to manage any data of the cartridge 202 in various ways. In some cases, the selection(s) received are utilized to identify, retrieve, generate, determine, alter, confirm, acknowledge, flag, store, modify, manage in any other way, or any combination thereof, any data of the cartridge 202.

In various examples, one or more processes based on the communication(s) and/or the selection(s) are utilized to identify, retrieve, generate, determine, alter, confirm, acknowledge, flag, store, modify, manage in any other way, or any combination thereof, various subsets of the cartridge data. The process(es), for instance, produce and/ generate identify results data based on request data. The request data includes, for example, retrieve request data, generate request data, determine request data, alter request data, confirm request data, acknowledge request data, flag request data, store request data, modify request data, or any combination thereof, which are identifiable as corresponding request based on completion of the corresponding process(es) performed for the corresponding selection(s)

The process(es), for instance, produce and/ generate identify results data, which may be included in the identifier response(s) based on the identifier request(s). The results data includes, for example, retrieve results data, generate results data, determine results data, alter results data, confirm results data, acknowledge results data, flag results data, store results data, modify results data, or any combination thereof, which are identifiable as corresponding results based on completion of the corresponding process(es) performed for the corresponding selection(s).

In various implementations, various types of communication(s), input, and/or output, and/or data being managed therewith, are associated with different levels of importance. For example, the output of the AED 204 being managed with different levels of importance includes visual and/or audible output. In some cases, various types of the output by the AED 204 require one or more corresponding responses. In some cases, the AED 204, based on the settings data, utilizes one or more default setting(s) to halt any operation(s) based on output being performed. In some cases, the AED 204, based on the settings data, utilizes one or more default setting(s) to halt based on output of one or more notifications that have a relatively higher level of priority. For example, the high priority notification(s) are utilized by the AED 204, based on the settings data, to halt until confirmation is received. The high priority notification(s), for example, including notifications with the usage data 118.

Although the various types of communication(s) of different levels of importance are managed in various ways, as discussed above in the current disclosure, it is not limited as such. In alternative or additional examples, the settings data is utilized to control output of the high priority notification(s). In some cases, the AED 204, based on the settings data, utilizes one or more default setting(s) to continue operation(s) and also, simultaneously, to continue output of the high priority notification(s) at a portion of the display that is not distracting. In some cases, the AED 204, based on the settings data, utilizes one or more default setting(s) to continue operation(s) and also, simultaneously, to continue output of the high priority notification(s) at an area of the display that does not interfere with output of any type related to normal operation(s). In some cases, the AED 204, based on the settings data, utilizes one or more default setting(s) to continue operation(s) and also, simultaneously, to continue output of the high priority notification(s) by illuminating an indicator and/or outputting an audible alert, and/or by managing output by the AED 204 in any other way, or any combination thereof.

In some examples, the notifications(s) include one or more identifier notifications, which include identifier notification data. In some cases, any of the identifier notification(s) are managed as one or more high priority notifications and/or normal priority notifications. For instance, the identifier notification(s) are output in various ways. For instance, the identifier notification(s) are transmitted and/or visually/audibly output via automated processes of the AED 204. In some examples, the identifier notification(s) are output (e.g., transmitted and/or visually/audibly output) without requiring subsequent user input. In those or other examples, the identifier notification(s), such as the normal priority notifications, are output without requiring subsequent user input in response. For instance, the identifier notification(s) are output based on various activations (e.g., initial activations), modifications, changes, updates, etc., to the cartridge 202 and/or the AED 204, and/or any data associated therewith. In various instances, one or more communications, and/or one or more selections via user input from the user(s), are received as the identifier request(s).

In some examples, one or more subsets of the cartridge data 116 are output in the usage notification(s) by the AED 204. For instance, the subset(s) of the cartridge data 116 being output in the usage notification(s) include the usage data 118.

In various instances, individual ones of the identifier notifications(s) are automated in response to various occurrences. For example, the identifier notifications(s) are automated based on the cartridge 202 being removably connected to the AED 204, power being supplied to the cartridge 202 and/or the AED 204, activation of the cartridge 202 and/or the AED 204, identification of replacement, alteration, and/or modification of any of the component(s) and/or the software subset(s) of the cartridge 202 and/or the AED 204, expiration of a time period (e.g., a difference between an initial date and a current date being greater than a threshold, the initial time including any of the date(s), as discussed above in further detail), or any combination thereof.

Alternatively or additionally, individual ones of the identifier notifications(s) are automated based on one or more corresponding operation related triggers (e.g., one or more predetermined, preprogrammed triggers, etc.). The triggers, for example, may be associated with corresponding operation(s) and/or corresponding process(es) of the cartridge 202 and/or the AED 204). For instance, at least one of any of the identifier notifications(s) is automated based on at least one of the operation(s) and/or process(es) of the cartridge 202 and/or the AED 204 beginning and/or completing, etc.

Alternatively or additionally, individual ones of the identifier notifications(s) are automated based on one or more communication and/or user input related triggers being set, the trigger(s) being set based on one or more corresponding types of communication(s) and/or user input. Alternatively or additionally, the trigger(s) include one or more triggers associated with one or more values of communication and/or user input matching one or more predetermined and/or preprogrammed values. Alternatively or additionally, at least one of any of the identifier notifications(s) is automated based on one or more other values of communication and/or user input exceeding one or more corresponding predetermined and/or preprogrammed threshold values.

Although the settings data may be managed in various ways based on the cartridge 202, and/or the sub-device(s) therein, not being new, as discussed above in the current disclosure, it is not limited as such. In various cases, the settings data is managed via the communication(s) and/or the user input based on the cartridge 202, and/or the sub-device(s) therein, not being new, to control the AED 204 to perform normal operations. For example, the AED 204 is managed to performs normal operations notwithstanding the usage data 118 indicating that based on the cartridge 202, and/or the sub-device(s) therein, are not new. In such an example, the AED 204 is managed to enable treatment which is possibly crucial and/or urgent, such as to enabling the AED 204 to be used in such occurrences where another cartridge unavailable or not easily obtainable. In those or other examples, any restriction(s) may be overridden and/or ignored (e.g., if enabled as such in the settings data) by the communication(s) and/or the selection(s) received via user input.

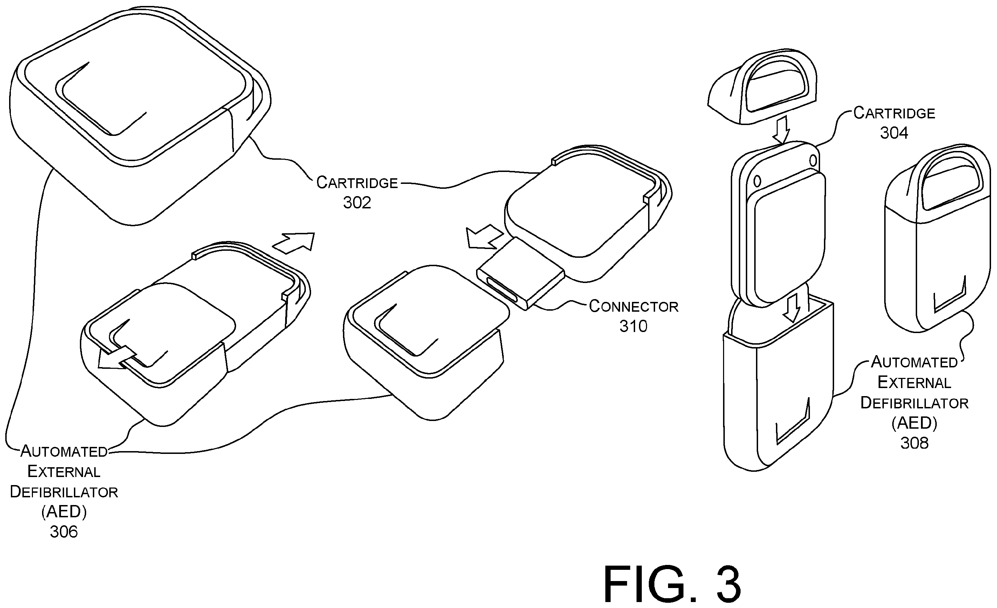

FIG. 3 illustrates example cartridges 302 and 304 and automated external defibrillators (AEDs) 306 and 308, the cartridges 302 and 304 configured to be removably coupled to the AEDs 306 and 308. For example, any of the cartridges 302 and 304, any of the AEDs 306 and 308 are utilized to implement the cartridge 102 and the AED 204, respectively, described above with reference to FIG. 1.

In some examples, the cartridge 302 is partially insertable into the AED 306, with a portion (e.g., a partial portion) of the cartridge 302 being external/outside the AED 306 (e.g., and accessible by a user while the AED 306 is in use). For instance, the cartridge 302 includes a handle that is usable by the user to insert and/or remove the cartridge 302. The cartridge 302, in some cases, is not fully enclosed by the AED 306.

In various cases, the cartridge 302 includes a connector (e.g., a male connector) 310 that is at least partially external to, and/or extends/protrudes from, a portion of the cartridge 302. For instance, the portion of the cartridge 302 includes a side, a case, a housing, a wall, etc., of the cartridge 302. The connector 310, for example, is attached to one or more wires, one or more cables, or one or more portions (e.g., one or more pieces of hardware) in the cartridge 302, and has one or more exposed, unshielded electrical terminals.

In some cases, one or more portions of the cartridge 302 and/or one or more portions of the AED 306 are connectable in a secure fashion. The portion(s) of the cartridge 302 and/or the portion(s) of the AED 306, for instance, are assembled with both portions being connected together to enclose (e.g., seal) the cartridge 302 to the AED 306. In some cases, individual ones of the portion(s) of the cartridge 302 and/or individual ones of the portion(s) of the AED 306 include one or more clips, one or more clasps, one or more straps, one or more lips, one or more hinges, one or more fasteners of any types, or any combination thereof, configured to connect the portions of the AED 306 together to form a watertight seal between the cartridge 302 and the AED 306.

In various examples, the AED 306 includes a cavity into which the cartridge 302 and/or the connector 310 is inserted. In some cases, the housing and/or the case of the AED 306 includes a cavity into which the housing and/or the case of the cartridge 304 and/or the connector 310 is inserted. For instance, the AED 306 includes, in the cavity, a connector (e.g., a female connector). In some examples, the connector in the AED 306 includes one or more terminals (e.g., one or more holes) to which the connector 310 exposed (e.g., a plug-type exposed conductor) is securely attached for a secure connection. For instance, one or more portions (e.g., a side, a case, a housing, a wall etc., of the cartridge 302) of the AED 308 intrudes within itself to form the terminal(s). The connector of the AED 306, for example, is attached to one or more wires, one or more cables, or one or more portions (e.g., pieces of hardware) in the AED.

In some implementations, the cartridge 304 (e.g., a housing and/or a case of the cartridge 304) is fully insertable into the AED 308 (e.g., a housing and/or a case of the AED 308). For instance, a portion (e.g., an entire portion) of the cartridge 302 is internal/inside the AED 308. In some cases, the entire portion of the cartridge 302 inaccessible, externally, such as by a user while the AED 308 is in use. For instance, the cartridge 304 does not include any handle. The cartridge 304, in some cases, is fully enclosed by the AED 308, which includes portions that are connectable to each other. For example, the portions of the AED 308 include a main (e.g., primary) portion (or "bottom") and an auxiliary (e.g., secondary) portion (or "top") that are attached to one another.

In some cases, the portions of the AED 308 are connectable in a secure fashion. The AED 308, for instance, is assembled with both portions being connected together to enclose (e.g., seal around) the cartridge 304. In some cases, individual ones of the portions of the AED 308 include one or more clips, one or more clasps, one or more straps, one or more lips, one or more hinges, one or more fasteners of any types, or any combination thereof, configured to connect the portions of the AED 308 together to form a watertight seal.

In various cases, the cartridge 304 includes a connector (e.g., a female connector) that includes one or more terminals (e.g., one or more holes) to which a connector of the AED 308 is exposed (e.g., a plug-type exposed conductor). For instance, the connector of the cartridge 304 is securely attached to the connector of the AED 308 for a secure connection. In some cases, the AED 308 includes a connector (e.g., a male connector) that is at least partially external to, and/or extends/protrudes from, a portion (e.g., a side, a portion of a case, a wall, etc.) of the AED 308. Individual ones of the connectors of the cartridge 304 and/or the AED 308, for example, are attached to one or more wires, one or more cables, or one or more portions (e.g., one or more pieces of hardware) in the cartridge 302.

In some implementations, the AEDs 306 and/or 308 are configured to receive sub-devices (e.g., removable sub-devices), which include the cartridges 302 and/or 304, respectively. In some cases, the sub-devices include one or more other types of sub-devices included the cartridges 302 and/or 304. In various cases, the sub-devices included in individual ones of the cartridges 302 and/or 304 include one or more power supplies, one or more electrode pads, one or more sensors, one or more of various other types of electrical devices, or any combination thereof.

Although various types of connectors may be included in the cartridges 302 and 304 and the AEDs 306 and 308, as discussed above in the current disclosure, it is not limited as such. In some examples, individual ones of the cartridges 302 and 304 include any connectors of any types, and/or individual ones of the AEDs 306 and 308 include any connectors of any types. In various cases, individual ones of the connectors include various portions, including one or more ports, one or more plugs, one or more pins, one or more sockets, one or more terminals, one or more of other types of connectors, or any combination thereof.

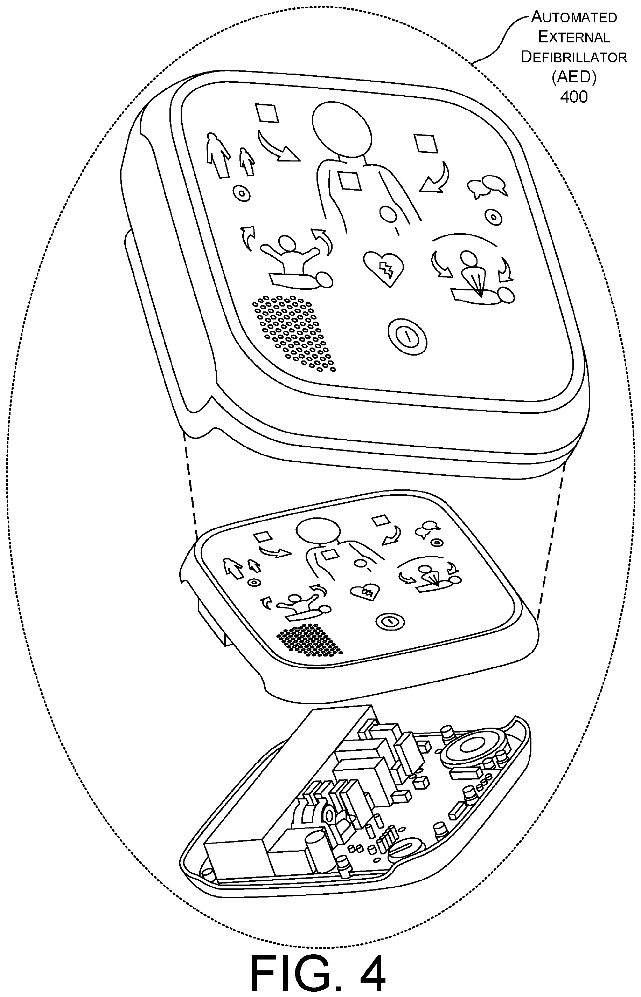

FIG. 4 an example automated external defibrillator (AED) 400 that is non-adhesively sealed and that is connectable to a removably couplable cartridge. For example, the AED 400 is utilized to implement the AED 104, described above with reference to FIG. 1.

In some examples, portions of the AED 400 are connectable to one another. For instance, individual ones of the portions of the AED 400 include one or more clips, one or more clasps, one or more straps, one or more lips, one or more hinges, one or more fasteners of any types, or any combination thereof, configured to connect the portions of the AED 400 together to form a watertight seal.

In various cases, the portions of the AED 400 are configured to fit together, thereby forming the watertight seal, without any adhesive. For example, the portions of the AED 400 are configured to fit and connect, by using an over-molded configuration (e.g., an over-molded plastic configuration). In some cases, the over-molded configuration of the AED 400 is based on performance, during manufacturing, of an injection molding process, allowing an additional layer of resin to be added to an existing molded part to provide a combination of characteristics (e.g., characteristics that would otherwise be unavailable by using a single material).

The watertight seal between the portions of the AED 400 is formed, for example, without any glue and/or any other type of adhesive. In some cases, the portions of the AED 400 are keyed to be assembled, and/or physical aligned to fit together, according to one orientation and/or physical configuration.

In some examples, the portions of the AED 400 are connected by one or more screws. For instance, the portions of the AED 400 include a printed circuit board (PCB) in the middle (e.g., center) of the portions (e.g., the PCB is sandwiched between the portions). In such an instance, the screw connects one or more surfaces associated with the corresponding portion(s) of the AED 400 by a seal that is non-adhesive and watertight.

Although the portions of any AED may be configured to fit together, thereby forming the watertight seal, without any adhesive, as discussed above in the current disclosure, it is not limited as such. In various examples, any of the portions of any AED (e.g., the AED 400) are configured to fit together, thereby forming any seal of any type (e.g., a partially watertight seal, a completely watertight seal, a water resistant seal, a non-water resistant seal, a non-watertight seal, etc.), with adhesive (e.g., an adhesive partially forming any seal, an adhesive completely forming any seal, etc.) between any portions of the AED 400, for purposes of implementing any techniques as discussed throughout the current disclosure.

In some examples, an individual, such as the patient 106, as discussed above with respect to FIG. 1, may be experiencing a medical emergency. For instance, the patient 106 may be treated by another individual, such as the user 112, as discussed above with respect to FIG. 1, which may be a random passerby (e.g., an unspecialized non-medical person). The user 112 may, for example, obtain the AED 400 for providing medical treatment to the patient 106. Because the user 112 may not be specially trained and/or experienced in providing medical treatment with AED 400, valuable instructions provided by the AED 400 may be decisive with respect to whether the user 112 is able to successfully medical treat the patient 106.

In those or other examples, the user 112 may activate and/or power on the AED 400, which reports out to the user 112 instructions on how to operate the AED 400. For instance, the AED 400 may output (e.g., visually present, by a display, and/or audibly output, by a speaker) information associated with the battery 108 and/or the electrode pads 110, as discussed above with reference to FIG. 1. In some cases, the information includes instructions for how the user 112 can check a charge level of the battery 108 and/or a usage state (or "usage status") of the electrode pads 110. In those or other examples, the AED 400 may output (e.g., visually present, by a display, and/or audibly output, by a speaker) an identifier representing the battery and information (e.g., text, audible words, etc., such as "New" and/or "Fully Charged," etc.). In various examples, the identifier includes a name, a symbol (e.g., a battery symbol), etc., or any combination thereof, representing the battery. In various examples, the identifier is output in various ways, such as with text, audible words (e.g., "New" and/or "Fully Charged"), etc.

In some examples, such as with the information being visually presented, the text is presented adjacent to the battery symbol. The AED 400 may present a symbol (e.g., an electrode pads symbol) representing the electrode pads 110, and text, such as "New," adjacent to the electrode pads symbol. The AED 400 may present the symbols and the texts based on retrieving the cartridge data 116, from the memory 114. For example, the cartridge data 116, including a value indicating that the battery 108 is fully charged, and a value indicating that the electrode pads 110 are new, is utilized by the AED 400 to present the symbols and the texts.

In those or other examples, after presenting the information indicating that the battery 108 has a full charge and that the electrode pads 110 are new, the AED 400 may output (e.g., visually present, by a display, and/or audibly output, by a speaker) additional instructions to the user 112. For example, the AED 400 may output (e.g., visually present, by a display, and/or audibly output, by a speaker) information informing the user how to place the electrode pads 110 on the patient 106, how to retrieve physiological data associated with the patient 106 and/or how to administer defibrillation to the patient 106.

By presenting the instructions on the display of the AED 400, and/or by audibly outputting the instructions by the speaker of the AED 400, a likelihood of the user 112 successfully medically treating the patient 106 may be significantly increased. The user 112 is able to obtain the instructions regarding usage of the AED 400 based on the cartridge data 116 stored in the cartridge 102, as discussed above with reference to FIG. 1. The likelihood of successful outcomes of the medical treatment being applied, even including the user 112 being able to save the life of the patient 106, is increased significantly by the presenting of the instructions by the AED 400.

The user 112, for example, obtains important compatibility related data indicating that the cartridge 102 is ready to be used quickly and "correctly" during an emergency scenario. The compatibility related data includes, for example, data that is permanently stored in the cartridge 102, in addition to the usage data 118, as discussed above with reference to FIG. 1, which modifiable. The various types of data may be output, for example, to raise a likelihood of a successful outcome of any medical treatment being applied. In such an example or another example, any cartridge and/or cartridge data associated therewith is automatically and/or manually updated based on the cartridge being used, to prevent any cartridge that is not new from being inadvertently used in the future without any user being aware of the previous use of the cartridge 102. In such an example or another example, any cartridge and/or cartridge data associated therewith is automatically and/or manually updated to prevent any cartridge that is relatively old, such as being past an expiration date, etc., from being inadvertently used. By reducing the likelihood of any used cartridge being inadvertently reused, or by, at a minimum, enabling users in the future to understand that the cartridge is relatively old and/or previously used, future usages of the cartridge that might otherwise be unsuccessful or suboptimal, due to the cartridge being relatively old and/or been previously used may be avoided.

Although the usage data 118 may be associated with the cartridge 102, and/or the sub-device(s) therein, as discussed above in the current disclosure, it is not limited as such. In some examples, the usage data 118 is updated based on any changes to any of one or more subsets of the cartridge data 116, as discussed above with reference to FIG. 1. The subset(s) include, for example, data being initially stored in the cartridge 102. For instance, any of the subset(s) of the cartridge data being initially stored is utilized to enable initial usage of the cartridge 102. For instance, the subset(s) of the cartridge data being initially stored is stored in the cartridge 102 during various processes prior to any utilization of the cartridge 102 by any user. In such an instance or another instance, the subset(s) of the cartridge data being initially stored is stored in the memory 114.

In some implementations, the subset(s) of the cartridge data being initially stored in the cartridge 102 are stored during various processes, which are performed prior to any user related processes being performed. For instance, individual ones of the subset(s) of the cartridge data being initially stored are stored during manufacturing, configuration, quality service review, production, shipping, storing, stocking, inventorying, operating, various other processes of various types, or any combination thereof.

In some implementations, the subset(s) of the cartridge data being initially stored in the cartridge 102 includes one or more subsets of various types. In various instances, the subset(s) include a partial set of permanent, unalterable, data (e.g., data hard-wired into the cartridge 102, and/or data stored and/or managed with a read-only permission). In various instances, the unalterable data is the same initially as at the end of the life of the cartridge 102. The unalterable data, for example, is managed (e.g., stored) separately, and differently from, the usage data 118 being managed as alterable data.

In some examples, one or more permissions (e.g., the read-only permission) are utilized to control access, and/or modification rights, associated with the unalterable data. By restricting operations of the cartridge 102 that would otherwise be utilized to modify the partial set of the cartridge data 110, the partial set of the cartridge data 110 is protected. By protecting the partial set of the cartridge data 110, the cartridge 102 is secured from unwanted tampering, unexpected alterations, and/or incidental operations, so that the cartridge 102 is verifiable as a correct type of cartridge, a compatible cartridge, a cartridge that meets one or more standards of manufacturing, production, design, maintenance, etc. By ensuring that important data is not tampered with and/or changed, the likelihood of the user 112 being able to medically treat the patient 106 successfully and confidently is improved.

In various instances, one or more subsets of the component data, as discussed above with reference to FIG. 1, are stored as the unalterable data. In those or other instances, the cartridge 102 is designed to include, for example, hardware and/or software that prevents modification of any of the component data that is unalterable. In those or other instances, the hardware and/or software preventing modification to the unalterable component data is utilized to ensure that the unalterable component data is not modified, restrict modifications to the unalterable component data, render the unalterable component data incapable of being modified, or any combination thereof.

In various examples, the subset(s) of the cartridge data 110 include restricted data, which may be separated out from the usage data 118. The restricted data, for example, is not able to be communicated. In some cases, the restricted data includes data that is unique to the cartridge 102, such as confidential and/or security data. For example, the restricted data includes the identifier(s) that are unique for the cartridge 102, and/or one or more portions of the cartridge data 110 that are not able to be communicated for security protection of the cartridge 102 and/or the AED 400, and/or that are not usable and/or of interest to one or more users of the external device(s). In some cases, the restricted data includes fully restricted data that is restricted from transfer to any of the external device(s) 120.

In those or other cases, the restricted data includes partially restricted data that is restricted from transfer to one or more unknown external devices but allowed for transmission to known external devices. For example, individual ones of the external device(s) 120 include the unknown external device(s) and/or the known external device(s). For example, individual ones of the external device(s) 120 are configured, preconfigured, designed, specialized, etc. as the unknown external device(s) and/or the known external device(s). In various instances, the known external device(s) include any types of devices with passwords establishing themselves with the AED 400 as known device. In some cases, the known external device(s) include software that communicates (e.g., possibly unbeknownst to user(s) of the known external devices and/or in the background) with the AED 400 to establish communications with the AED 400 automatically based on one or more requests being transmitted by the AED 400 and to the known external device, or vice versa.

In some cases, the unknown external device(s) are not been authorized by the AED 400 for communication. For example, the unknown external device(s) transmit requests to the AED 400 for communication and/or that respond to one or more authorization notifications transmitted by the by the AED 400. In some cases, the AED 400 transits the authorization notifications in response to receiving one or more requests from the unknown external device(s). In some instances, an unknown external device transmits a connection request that is processed by the AED 400 and utilized to successfully verify the unknown external device as a known external device based on the unknown external device providing confirmation (e.g., confirmation via a password and/or security key, such as via an authorization request to the AED 400), which is utilized by the AED 400 to authenticate the unknown external device authorized for communication with the AED 400.

In various implementations, an unknown external device transmits a connection request that is processed by the AED 400 and not successfully utilized to verify the unknown external device based on the unknown external device not providing confirmation (e.g., confirmation via a password and/or security key) that the unknown external device is authorized for communication with the AED 400. Alternatively or additionally, the AED 400 identifies the unknown external device as not being a known external device based on an omission of any authorization request from the unknown external device in response to receiving the authorization notifications from the AED 400.

In various implementations, one or more communications are exchanged with an unknown external device via a first communication protocol to perform authentication. In some cases, one or more communications are exchanged, based on the unknown external device being verified as a known external device, with the known external device using a second communication protocol. For example, the first communication protocol includes the NFC protocol, and the second communication protocol includes a wireless protocol (e.g., cellular protocol) and/or the bluetooth protocol.

Although the first communication protocol and the second communication protocol may include types of communication protocols, as discussed above in the current disclosure, it is not limited as such. In various examples, the first communication protocol, the communication protocol, one or more other communication protocols associated with any communications are utilized in a similar way as the first and/or second communication protocols for purposes of implementing any of the techniques discussed herein.

By utilizing authentication communications before enabling communications with an unknown device, the AED 400 ensures that only secure devices are able to establish communications. By restricting the communication(s) to allow only the communication(s) with the known external devices, in some cases, one or more other communications that might needlessly and/or dangerously (e.g., dangerously with respect to being not being able to property operate for medical treatment) take up bandwidth and/or network/processing resources of the AED 400 may be minimized and/or prevented.

In various examples, the usage data 118 is communicated using the same protocols as one or more other types of the cartridge data 116. In other examples, the usage data 118 is communicated using different protocols from one or more other types of the cartridge data 116. In some cases, the usage data 118 is communicated using different protocols from all other types of the cartridge data 116.

Although the AED 400 and the external device may perform authentication communications, as discussed above in the current disclosure, it is not limited as such. In various examples, the AED 400 is utilized to communicate with the external device(s) 120 without performing authentication (e.g., without exchanging the authentication communications) (e.g., regardless of the external device(s) being known or unknown). For example, the AED 400 and the external device exchange communication(s) via any of the communication protocol(s) based on one or more notifications (e.g., one or more periodically transmitted notifications, such as one or more pings, etc.) transmitted by the AED 400 and to the external device(s) 120, and/or based on one or more requests transmitted by the external device(s) 120 and to the AED 400.

By enabling communication between the AED 400 and/or the external device(s) 120 without authentication processes/communications, any data that may be important to medical treatment may be provided by the user 112 and/or any other user (e.g., a medical technician, a physician, a paramedic, an emergency medical technician (EMT), any other type of user, or any combination thereof), without interruptions, delays, conflicts, and/or failures in communicating the data.

In various cases, the AED 400 transmits one or more notifications (e.g., one or more notification signals) at any time, triggering one or more notifications (e.g., one or more prompts and/or indications) to be displayed by the external device(s) 120. For instance, the prompts and/or indications include text indicating any portions of the usage data 118. In some cases, the prompts and/or indications include text indicating notification(s) are associated with the AED 400. The text includes any of the identifier(s) of the AED 400 and/or text with a generic description, such as "AED Data." In some cases, one or more subsets of software (e.g., one or more applications, one or more programs, etc.) of any type being executed by the external device(s) 120 are utilized to process the notification(s), to receive and/or to store the subset(s) of the cartridge data 116, and/or any sensor data associated with operation of the cartridge 102 and/or the AED 400, and/or to present via one or more displays of the external device(s) 120, the received and/or stored data.

In various cases, the subset(s) of the cartridge data being initially stored in the cartridge 102 also possibly includes a partial set that is modifiable, able to be enhanced, expandable, redactable, alterable in any other way. The partial set, for instance, partial set that is alterable includes some, or possibly all, of the cartridge data 116. In such an example or another example, the partial set of the cartridge data 116 that is alterable includes nonpermanent, alterable data. The alterable data, for instance, is stored and/or managed with a read-write permission.

In some cases, updating of the partial set of cartridge data 116 that is alterable is automated. For example, hardware and/or software of the cartridge 102 automatically updates the cartridge data 110 at any time (e.g., prior to, during, or after usage, as discussed above in further detail), the time being designated, predetermined, preprogrammed, any other time of any type, or any combination thereof.

In various examples, the alterable data includes any of one or more subsets of data with various types of permissions (e.g., write permissions), alternatively or in addition to, read-only permissions, and/or any subsets of data that are alterable by one or more operations of the cartridge 102 and/or the AED 400. In some examples, any of the hardware and/or software of the cartridge 102 and/or the AED 400 may be utilized to enable the subset(s) of data from being altered and to manage the subset(s) of data as the alterable data

In some instances, the cartridge data 116 includes data indicating that the alterable data has been altered. For example, the cartridge data 116 includes one or more flags indicating that the alterable data has been modified, enhanced, expanded, redacted, or altered in any other way, or any combination thereof. In some cases, one or more flags and/or identifiers included in the cartridge data 116 indicate that corresponding subset(s) of the alterable data have been altered. For instance, the flag(s) and/or the identifier(s) being output are utilized to indicate the data having been altered. In some cases, the identifier(s) possibly include previous and/or current corresponding values of the data based on the altering having been performed. The flag(s) and/or the identifier(s), in some examples, are communicated and/or visually/audibly output via one or more notifications. The flag(s) and/or the identifier(s) are set and/or updated, for example, based on an AED/cartridge activation, an AED/cartridge power-on event, triggering by various types of user input, triggering at periodic times, triggering by various types of communications, etc., or any combination thereof.

Although various sets/subsets, and/or partial sets/subsets, of the cartridge data 116 may be unalterable and/or various sets/subsets, and/or partial sets/subsets, of the cartridge data 116 may be alterable, as discussed above in the current disclosure, it is not limited as such. In some examples, at least one of the various sets/subsets and/or partial sets/subsets of the cartridge data 116 that are unalterable are changeable to be alterable, and/or vice versa. For example, any data being changed from alterable to unalterable, and/or vice versa (e.g., and stored accordingly), is managed in various ways, such as based on changes to the setting(s) of the cartridge 102 and/or the AED 400, as discussed below in further detail. In some instances, any type of initial data (e.g., the initial setting(s) set prior to any usage) is stored by the cartridge 102 and/or the AED 400 to manage any type of any data of the cartridge 102 and/or the AED 400 as alterable or unalterable.

In various implementations, automated management of any of the cartridge data 116 being stored as alterable data includes altering of any type, such as modifying (e.g., changing any of the alterable data), enhancing, expanding, redacting, or altering data in any other way, or any combination thereof. The enhancing includes, for example, changing from using one type of data management to another that is more thorough and/or detailed, such as from managing tic marks of usages to managing numbers of usages, also, etc. The expanding incudes, for example, changing from one type of management to another, such as from managing usages associated with only the cartridge 102, to managing usages associated with various sub-devices, also. The redacting includes, for example, removing any type of management based on changes to the setting(s), such as by removing managing of numbers of usages, and subsequently managing only tic marks of usages.

In various implementations, the settings data may include override settings to change the unalterable data to be alterable data, or vice versa. For example, management of the cartridge data 116 may include unalterable data being changed to be alterable data, or vice versa. For example, unalterable data being changed to be alterable data, or vice versa, may be performed via the settings data. overridden includes enabling overriding.

In some instances, one or more overrides are performed via the update(s) for any of the settings data. For example, the update(s) include setting, and/or changing, any of one or more overrides in the settings data. In such an example, one or more subsets of the cartridge data 116 that are unalterable are overridden to be alterable, or vice versa. The overrides are performed, for instance, based on one or more confirmations received from the external device(s) 120, and/or via one or more selections received via user input. For instance, one or more unalterable states for the subset(s) of the cartridge data 116 are overridden in response to corresponding notification(s) that are output (e.g., communicated and/or visually/audibly output) indicating that the data was marked as unalterable.

Although the usage data 118 may be managed as the alterable data, as discussed above in the current disclosure, it is not limited as such. In some examples, the usage data 118, being managed as the alterable data, may be managed as temporary data. For instance, the usage data 118 being managed as the temporary data may be stored and not altered for a period of time from an initial time. In some cases, the usage data 118 may be stored and not altered for the period of time being less than a period of time during which the permanent data is stored and not altered. In various examples, the usage data 118 may be stored and not altered for the period of time being less than a threshold period of time.

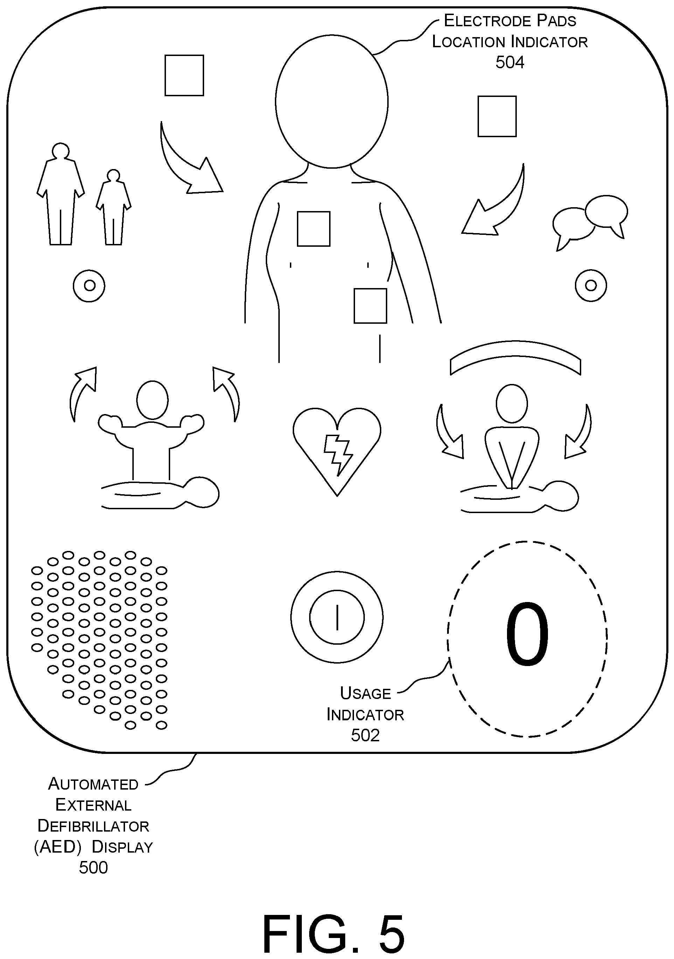

FIG. 5 illustrates an example display 500 of an automated external defibrillator (AED), the display 500 including a usage indicator 502 associated with usage of a cartridge that is removably coupled to the AED. For example, the display (or "AED display") 500 is utilized to implement any of the displays of the AEDs 104, 204, 306, and/or 308 described above with reference to FIGS. 1-4.

In some examples, the display 500 includes the usage indicator 502 (e.g., an indicator from among the indicator(s) as discussed above with reference to FIG. 1) and utilizes the usage indicator 502 to present a usage value in usage data (e.g., the usage data 118). For example, the display 500 presents, based on the AED being activated (e.g., connected to a battery, and/or to the cartridge that includes a battery) and/or being powered-on (e.g., via activation of a power button, switch, slide, toggle, any of other types of activation devices, or any combination thereof), the usage value.

In some cases, the display 500 presents the usage value based on the AED identify a number of uses of the cartridge. For instance, such as with the cartridge being new, the display 500 presents the usage value as a numerical value of "0," as represented by the "0" being presented in the dotted-line oval of the illustration in FIG. 5.

In some cases, the AED identifies the usage value in various ways (e.g., any of the process(es) and/or operation(s) discussed above with reference to FIG. 1). For instance, the AED senses the cartridge being removably connected to the medical device and performs one or more operations to retrieve the cartridge data 116 and/or the usage value in the usage data 118. In some examples, the operation(s) utilized to retrieve the cartridge data 116 and/or the usage value are automated, in response to the sensing by the AED of the cartridge being removably connected.

In various implementations the AED is utilized to treat a patient and updates the cartridge data 116, accordingly. For example, the AED transmits (e.g., routes) a signal to the cartridge to update the cartridge data 116 and/or the cartridge, the usage value in the cartridge being replaced by a numerical value of "1." In such an example or another example, the AED presents the numerical value of "1" instead of "0," in response to the updating of the cartridge data 116. For instance, the AED presents the usage value as the numerical value of "1" while the cartridge is removably connected, in response to sensing the cartridge being removed, in response to sensing the same cartridge being removably connected again, in response to the AED being turned on and/or activated, any of various other processes triggering the presenting of the cartridge data 116 and/or the usage value, or any combination thereof.

In some cases, the AED and/or the cartridge performs regular tests and/or self-checks. For example, the AED and/or the cartridge performs a test (or "initial test") and/or a self-check (or "initial self-check") at an initial power-on and/or initial activation, and/or based on one or more selections via user input, instructing the AED and/or the cartridge to perform the test and/or the self-check. In such an example or another example, the AED and/or the cartridge identifies that a current date and/or a current time are one day (e.g., 24 hours) from the initial date and/or the initial time utilized for the test and/or self-check. In those or other examples, the AED and/or the cartridge identifies perform a test and/or a self-check in response to the identifying that the current date and/or the current time are one day (e.g., 24 hours) from the initial date and/or the initial time utilized for the test and/or the self-check.

In various examples, the test and/or the self-check is utilized to check an age of the cartridge 102. For example, the age represents one or more periods of time (e.g., a period of time between a current date and/or time and an expiration date and/or time) until the cartridge 102 expires. In some cases, identifying the age is performed by comparing a current date and/or time, etc., to an expiration date and/or time stored in the cartridge data, and identifying that the current date and/or time exceeds the expiration date and/or time.

In response to identifying (or "recognizing") that the current date and/or time exceeds the expiration date and/or time (e.g., recognizing that the age associated with the current date and/or time exceeds a threshold age, which includes a difference between an initial date and/or time at the initial activation, etc., and an expiration date and/or time), the AED outputs any of the notification(s) (e.g., an alert). In various cases, identifying whether the current date and/or time exceeds the expiration date and/or time is performed in various ways, such as at the trigger(s) activation(s), power-on(s), etc., utilized for the notification(s) of any type.

In some examples, the AED identifies that a current date (e.g., a new current date) and/or a current time (e.g., a new current time) are one day (e.g., 24 hours) from a previous date (e.g., the previous "current date" and/or any other previous date) and/or a previous time (e.g., the previous "current time" and/or any other previous time) utilized for the test and/or self-check. In those or other examples, the AED performs a test and/or a self-check in response to the identifying that the current date and/or the current time are one day (e.g., 24 hours) from the previous date and/or the previous time utilized for the test and/or the self-check.

Although the test and/or the self-check may be performed based on the one day and/or 24 hours period, as discussed above in the current disclosure, it is not limited as such. In some cases, the test and/or the self-check are performed based on any date and/or any time, and/or any type of trigger, which is configurable using settings data (e.g., the settings data as discussed above with respect to FIG. 1.

In various cases, the display 500 is utilized by the AED to present one or more indicators, including an electrode pads location indicator 504, symbolizing one or more locations of electrode pads (e.g., the electrode pads 110) on a patient. In some examples, the electrode pads location indicator 504 includes one or more portions (e.g., one or more geometric object shaped portions, including one or more square portions, one or more rectangle portions, any of one or more other portions of other shapes, or any combination thereof).

In some instances, the electrode pads location indicator 504 is identified, determined, selected, modified, etc., or any combination thereof, from among one or more types of indicators. For example, the electrode pads location indicator 504 includes an adult indicator,, a child indicator, a pediatric indicator (or "infant indicator"), or any combination thereof. In some cases, the adult indicator includes individual ones of the portion(s) representing one or more electrode pad placement locations on an adult patient (e.g., a patient of an age above a threshold adult age), the child indicator includes individual ones of the portion(s) representing one or more electrode pad placement locations on a child patient (e.g., a patient of an age below a threshold adult age and above a threshold pediatric age), and/or the pediatric indicator includes individual ones of the portion(s) representing one or more electrode pad placement on a pediatric patient (e.g., a patient of an age below a threshold pediatric age).

In some examples, individual ones of one or more groups of the location(s) associated with the adult, the child, and/or the infant include different locations. For instance, the location(s) associated with the adult and/or the child include a location at a right side of a chest, just below a collarbone, and another location at a lower left side of the chest. In such an instance or another instance, the location(s) associated with the infant include one location at a center of a chest of the patient, on a sternum, and a location at a back of the patient between the scapulae.

In various cases, input/output operations (e.g., any of the input/output operation(s) as discussed above with reference to FIG. 1) are utilized to obtain data regarding an age (e.g., an age in compliance with the thresholds for the adult age, an age in compliance with the thresholds for the child age, an age in compliance with the thresholds for the pediatric age, etc.) and/or a age category (e.g., adult, child, pediatric, etc.) of the patient. The operation(s) are performed, for example, at activation and/or power-on. The display presents one or more indications representing one or more inquiries for the age and/or age category of the patient.

In some examples, the AED presents, as the electrode pads location indicator 504, the adult indicator, the child indicator, or the pediatric indicator. The electrode pads location indicator 504, for instance, is presented as the adult indicator, the child indicator, or the pediatric indicator in response to receiving one or more selections via user input indicating the age and/or the age category of the patient.

Although one or more age ranges and/or the age category(ies) including adult, child, and/or pediatric may be utilized as discussed above in the current disclosure, it is not limited as such. In some cases, the age range(s) and/or the age category(ies) include any number of ages and/or age categories. For instance, the age(s) include only a non-pediatric age range and a pediatric age range, and/or the age category(ies) include only a non-pediatric age category and a pediatric age category. In those or other examples, the pediatric indicator is illuminate to represent the cartridge 102 and/or the AED being activated and/or energized for usage (e.g.., usage for outputting voltages from the electrode pads 110 at corresponding values below child threshold values) according to the pediatric age range and/or the pediatric age category.

In some examples, the age range(s) include a non-child age range and a child age range, and/or the age category(ies) include only a non-child age category and a child age category. In those or other examples, the child indicator is illuminate to represent the cartridge and/or the AED being activated and/or energized for usage (e.g.., usage for outputting voltages from the electrode pads 110 at corresponding values below threshold adult values and/or above threshold pediatric values) according to the child age range and/or the child age category.

Although the usage indicator 502 and/or the electrode pads location indicator 504 are utilized in various ways as discussed above in the current disclosure, it is not limited as such. In some examples, the usage indicator 502 and/or the electrode pads location indicator 504 are managed automatically based on the sensor data, as discussed above with reference to FIGS. 1 and 2. In those or other examples, the usage indicator 502 and/or the electrode pads location indicator 504 are managed automatically based on the settings data, the identifier data, and/or any other types of data, including the cartridge data 116, as discussed above with reference to FIGS. 1 and 2.

In some implementations, output of the AED 104, for example, regarding the cartridge 102 being new and/or unused is used as an assurance to the user 112 of operational readiness of the cartridge 102 and/or the AED 104. In contrast to existing medical devices which do not provide any usage information regarding removable cartridges, have a relatively greater likelihood of failing, and are unable to provide any assurance to users for verifying statuses, including usage statues, of removable cartridges, the cartridges and the medical devices implemented according to the techniques discussed herein, including the cartridge 102 and the AED 104, enable the user 112 to treat the patient 106 with the cartridge 102 and the AED 104, in response to verifying that the cartridge 102 and the AED 104 are in correct working order.

In some examples, the usage statuses in the usage data 118 include usage statuses of the cartridge 102 itself, and/or any usage statuses of any devices therein. For example, the usage data 118, enables the AED 104 to report out (e.g., communicate, present, indicate visually and/or audibly, etc., or any combination thereof) that all sub-devices of the cartridge 102 are fully operational. Information reported to the user by the AED 104 may include information indicating that the cartridge 102 is fully operational, including the battery 108 being fully operational (e.g., the battery 108 being fully charged), and the electrode pads 110 being fully operational (e.g., the electrode pads 110 being unused). A simple indicating, for example, may be presented by the AED 104, based on the cartridge data 110, to enable the user 112 to verify at a quick glance that the AED 104 is ready to be used.

In those or other examples, the AED 104 may display text of "Charged and Unused," indicating to the user 112 that the cartridge 102 is charged and unused (e.g., including that the battery 108 is charged and that the electrode pads 110 are unused). The output (e.g., the text), for instance, enables the user 112 to verify that the AED 104 is charged and unused, and may, in some cases, enable the user 112 to treat the patient 106 with proper speed and accuracy for responding to a medical emergency. In some cases, being able to verify, via the information output by the AED 104, that the cartridge 102 is charged and unused, may enable the user 112 to perform life saving treatment on the patient 106.

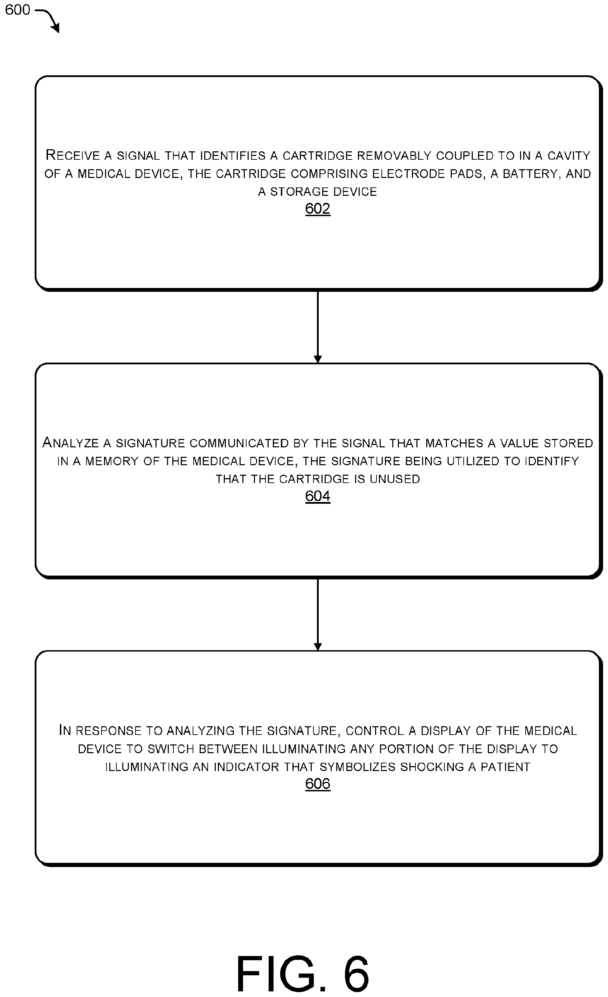

FIG. 6 illustrates example process 600 for utilizing a cartridge 102 that is removably couplable to an automated external defibrillator (AED) 104 utilized to monitor and treat a condition of a patient 106, the cartridge 102 including memory 114 utilized to store cartridge data 116 associated with the cartridge 202, as discussed above with reference to FIGS. 1-5.

At 602, the AED 104 receives a signal that identifies a cartridge 102 removably coupled to in a cavity of the AED 104, the cartridge 102 comprising electrode pads 110, a battery 108, and a storage device (e.g., a memory 114). In some examples, the AED 104, in which the cartridge is removably coupled, is utilized to treat a patient 106. For example, the treatment includes pace pulses, an electrical shock (e.g., a defibrillation treatment), mechanical chest compressions, assisted ventilation, or any combination thereof.

In some cases, the treatment includes identifying, based on one or more selections received via user input, confirming that the electrode pads 110 are positioned in correct locations. The treatment includes, for example, setting a flag identifying the electrode pads 110 are positioned in correct locations (e.g., that a first electrode pad is positioned on a chest located on the patient 106 and a second electrode pad is positioned on a midsection located on the patient 106).

In some examples, the treatment includes identifying the setting of the flag that identifies the first electrode pad being positioned on the chest located on the patient 106 and the second electrode pad being positioned on a midsection located on the patient 106. In those or other examples, the treatment includes identifying an electrocardiogram (ECG) characteristic in response to the setting of the flag and/or the identifying of the setting of the flag. In those or other examples, the treatment includes analyzing an amplitude represented by the ECG characteristic, and confirming the amplitude is greater than a threshold. In those or other examples, the treatment includes controlling the display to illuminate an indicator in response to confirming the amplitude is greater than the threshold, the indicator being utilized by the user 112 to output electric energy through the electrode pads 110.

At 604, the AED 104 analyzes a signature communicated by the signal that matches a value stored in a memory of the AED 104, the signature being utilized to identify that the cartridge 102 is unused (e.g., recognize an unused status). A usage value is identified based on the signature and utilized to illuminate a usage indicator.

At 606, the AED 104 in response to analyzing the signature, controls a display of the AED 104 to switch between illuminating any portion of the display to illuminating an indicator that symbolizes shocking a patient. The usage indicator, for example, is illuminated as a numeral "0" based on the cartridge 102 being unused.

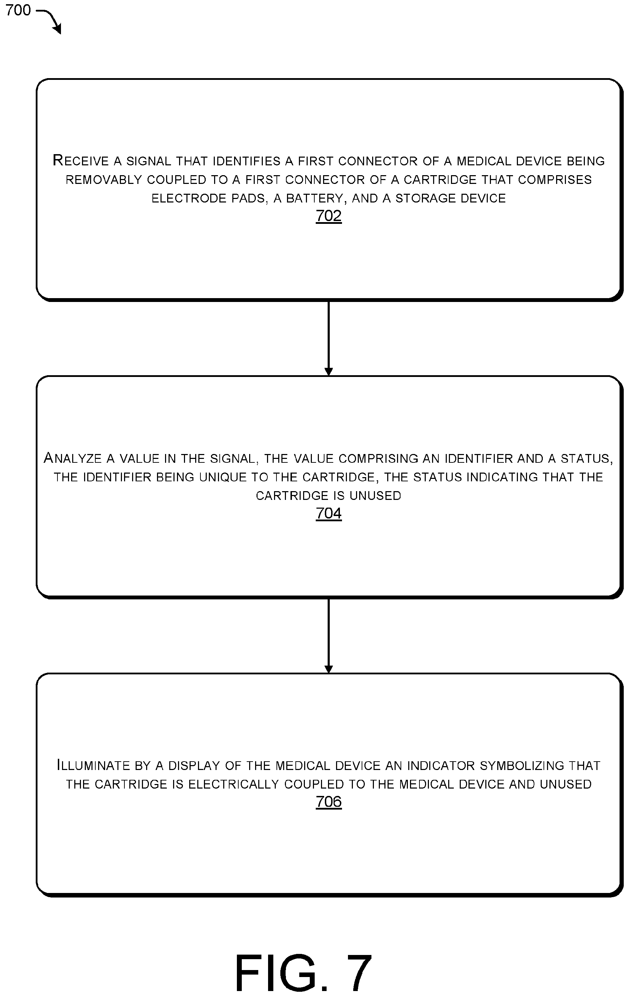

FIG. 7 illustrates example process 700 for utilizing a cartridge that is removably couplable to an automated external defibrillator (AED) utilized to monitor and treat a condition of a patient, the cartridge including memory utilized to store usage data associated with the cartridge, as discussed above with reference to FIGS. 1-5.

At 702, the AED 104 receives a signal that identifies a first connector of a AED 104 being removably coupled to a first connector of a cartridge 102 that comprises electrode pads 110, a battery 108, and a storage device (e.g., a memory 114). In some examples, the first connector includes a male or a female connector.

At 704, the AED 104 analyzes a value in the signal, the value comprising an identifier and a status, the identifier being unique to the cartridge 102, the status indicating that the cartridge 102 is unused. In various cases, the status of the cartridge 102 is identified via a usage value.

At 706, the AED 104 illuminates by a display of the AED 104 an indicator (e.g., a usage indicator 502) symbolizing that the cartridge 102 is electrically coupled to the AED 104 and unused. The cartridge 102 illuminates the usage indicator 502 in response to identifying the AED 104 is powered-on.

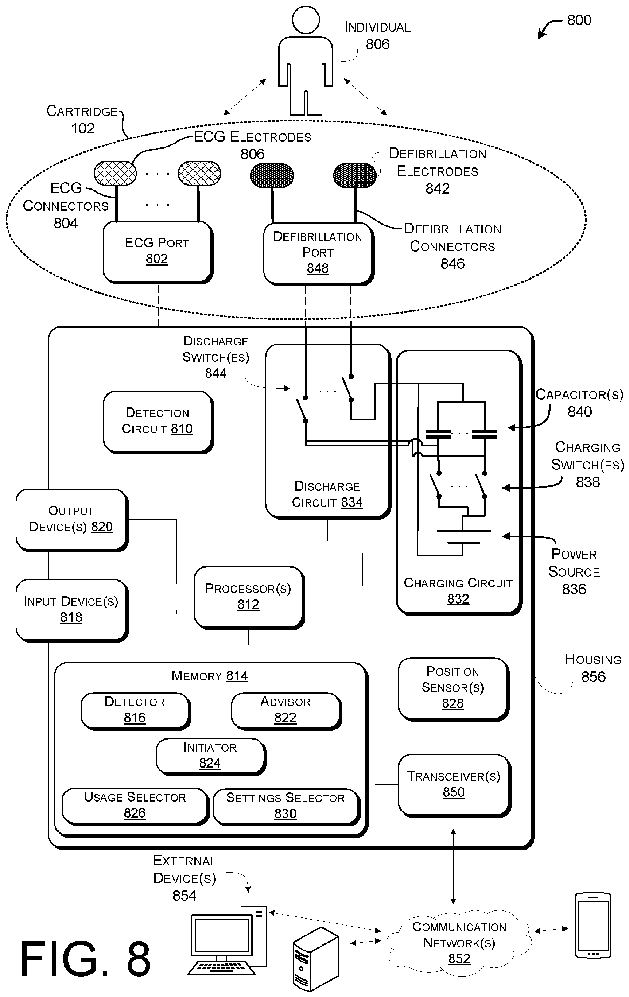

FIG. 8 illustrates an example of an external defibrillator 800 configured to perform various functions described herein. For example, the external defibrillator 800 is utilized to implement the cartridge 102, the automated external defibrillator (AED) 104, or any combination thereof, described above with reference to FIGS. 1 to 7.

The external defibrillator 800 is removably coupled to a cartridge (e.g., the cartridge 102), which includes an electrocardiogram (ECG) port 802 connected to multiple ECG connectors 804. In some cases, the ECG connectors 804 are removeable from the ECG port 802. For instance, the ECG connectors 804 are plugged into the ECG port 802. The ECG connectors 804 are connected to ECG electrodes 806, respectively. In various implementations, the ECG electrodes 806 are disposed on different locations on an individual 808. A detection circuit 810 is configured to detect relative voltages between the ECG electrodes 806. These voltages are indicative of the electrical activity of the individual's 808 heart. In various examples, individual ones of the ECG connectors 804 and/or individual ones of the ECG electrodes 806 are utilized to implement, individually or in combination, any corresponding electrode pads of the electrode pads 110, as discussed above with reference to FIG. 1.

In various implementations, the ECG electrodes 806 are in contact with the different locations on the individual's 808 skin. In some examples, a first one of the ECG electrodes 806 is placed on the skin between the heart and right arm of the individual 808, a second one of the ECG electrodes 806 is placed on the skin between the heart and left arm of the individual 808, and a third one of the ECG electrodes 806 is placed on the skin between the heart and a leg (either the left leg or the right leg) of the individual 808. In these examples, the detection circuit 808 is configured to measure the relative voltages between the first, second, and third ECG electrodes 806. Respective pairings of the ECG electrodes 806 are referred to as leads, and the voltages between the pairs of ECG electrodes 806 are known as lead voltages. In some examples, more than three ECG electrodes 806 are included, such that 5-lead or 12-lead ECG signals are detected by the detection circuit 810.

The detection circuit 810 includes at least one analog circuit, at least one digital circuit, or a combination thereof. The detection circuit 810 receives the analog electrical signals from the ECG electrodes 806, via the ECG port 802 and the ECG leads 804. In some cases, the detection circuit 810 includes one or more analog filters configured to filter noise and/or artifact from the electrical signals. The detection circuit 810 includes an analog-to-digital (ADC) in various examples. The detection circuit 810 generates a digital signal indicative of the analog electrical signals from the ECG electrodes 806. This digital signal can be referred to as an "ECG signal" or an "ECG."

In some cases, the detection circuit 810 further detects an electrical impedance between at least one pair of the ECG electrodes 806. For example, the detection circuit 810 includes, or otherwise controls, a power source that applies a known voltage across a pair of the ECG electrodes 806 and detects a resultant current between the pair of the ECG electrodes 806. The impedance is generated based on the applied voltage and the resultant current. In various cases, the impedance corresponds to respiration of the individual 808, chest compressions performed on the individual 808, and other physiological states of the individual 808. In various examples, the detection circuit 810 includes one or more analog filters configured to filter noise and/or artifact from the resultant current. The detection circuit 810 generates a digital signal indicative of the impedance using an ADC. This digital signal can be referred to as an "impedance signal" or an "impedance."

The detection circuit 810 provides the ECG signal and/or the impedance signal one or more processors 812 in the external defibrillator 800. In some implementations, the processor(s) 812 includes a central processing unit (CPU), a graphics processing unit (GPU), both CPU and GPU, or other processing unit or component known in the art.

The processor(s) 812 is operably connected to memory 814. In various implementations, the memory 814 is volatile (such as random-access memory (RAM)), non-volatile (such as read only memory (ROM), flash memory, etc.) or some combination of the two. The memory 814 stores instructions that, when executed by the processor(s) 812, causes the processor(s) 812 to perform various operations. In various examples, the memory 814 stores methods, threads, processes, applications, objects, modules, any other sort of executable instruction, or a combination thereof. In some cases, the memory 814 stores files, databases, or a combination thereof. In some examples, the memory 814 includes RAM, ROM, electrically erasable programmable read-only memory (EEPROM), flash memory, or any other memory technology. In some examples, the memory 814 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 812 and/or the external defibrillator 800. In some cases, the memory 814 at least temporarily stores the ECG signal and/or the impedance signal.

In various examples, the memory 814 includes a detector 816, which causes the processor(s) 812 to determine, based on the ECG signal and/or the impedance signal, whether the individual 808 is exhibiting a particular heart rhythm. For instance, the processor(s) 812 determines whether the individual 808 is experiencing a shockable rhythm that is treatable by defibrillation. Examples of shockable rhythms include ventricular fibrillation (VF) and pulseless ventricular tachycardia (V-Tach). In some examples, the processor(s) 812 determines whether any of a variety of different rhythms (e.g., asystole, sinus rhythm, atrial fibrillation (AF), etc.) are present in the ECG signal.

In some implementations, the processor(s) 812 is operably connected to one or more input/output devices. For instance, the input/output devices include one or more input devices 818, one or more output devices 820, one or more other input/output devices, or any combination thereof. Collectively, the input device(s) 818 and the output device(s) 820 function as an interface between a user and the defibrillator 800. The input device(s) 818 is configured to receive an input (e.g., any user input as discussed above with reference to FIGS. 1-7) from a user and includes at least one of a keypad, a cursor control, a touch-sensitive display, a voice input device (e.g., a speaker), a haptic feedback device, or any combination thereof. The output device(s) 820 is utilized for any output (e.g., any output as discussed above with reference to FIGS. 1-7) of the external defibrillator 800.

For example, the output device(s) 820 includes at least one of a display, a speaker, a haptic output device, a printer, one or more indicators (e.g., one or more visual indicator devices), or any combination thereof. In some examples, the indicator(s) include one or more liquid crystal display (LCD) indicators, one or more light emitting diode (LED) indicators, one or more organic light-emitting diode (OLED) indicators, one or more of any other types of indicators, or any combination thereof.

In various examples, the processor(s) 812 causes a display among the input/output device(s) (e.g., the input device(s) 818) to visually output a waveform of the ECG signal and/or the impedance signal. In some implementations, the input device(s) 818 includes one or more touch sensors (e.g., one or more touch sensors as part of a touchscreen, one or more touch sensors (e.g., capacitive, electromagnetic, etc., touch sensor(s)) on any portion(s) of the AED 104 (e.g., on a handle, any part of the housing, etc.), etc., or any combination there), the input/output device(s) (e.g., the output device(s) 820) includes a display screen, and the touch sensor(s) are integrated with the display screen. Thus, in some cases, the external defibrillator 800 includes a touchscreen configured to receive user input signal(s) and visually output physiological parameters, such as the ECG signal and/or the impedance signal.

In some examples, the memory 814 includes an advisor 822, which, when executed by the processor(s) 812, causes the processor(s) 812 to generate advice and/or control the output device(s) 820 to output the advice to a user (e.g., a rescuer). In some examples, the processor(s) 812 provides, or causes the output device(s) 820 to provide, an instruction to perform CPR on the individual 808. In some cases, the processor(s) 812 evaluates, based on the ECG signal, the impedance signal, or other physiological parameters, CPR being performed on the individual 808 and causes the output device(s) 820 to provide feedback about the CPR in the instruction. According to some examples, the processor(s) 812, upon identifying that a shockable rhythm is present in the ECG signal, causes the output device(s) 820 to output an instruction and/or recommendation to administer a defibrillation shock to the individual 808.

The memory 814 also includes an initiator 824 which, when executed by the processor(s) 812, causes the processor(s) 812 to control other elements of the external defibrillator 800 in order to administer a defibrillation shock to the individual 808. In some examples, the processor(s) 812 executing the initiator 824 selectively causes the administration of the defibrillation shock based on determining that the individual 808 is exhibiting the shockable rhythm and/or based on an input from a user (received, e.g., by the input/output device(s) (e.g., the input device(s) 818)). In some cases, the processor(s) 812 causes the defibrillation shock to be output at a particular time, which is determined by the processor(s) 812 based on the ECG signal and/or the impedance signal.

In various cases, the memory 814 includes a usage selector 826 which, when executed by the processor(s) 812, causes the processor(s) 812 to identify the usage data 118 based on an input signal received by the input device(s) 818. For example, a touchscreen of the input/output device(s) (e.g., the input device(s) 818) receives a signal indicative of the cartridge data 116. In some cases, a microphone of the input/output device(s) (e.g., the input device(s) 818) receives a signal to identify the usage data 118 based on an audible command (e.g., one or more utterances of a user). For example, the usage selector 826 causes the processor(s) 812 to identify the usage data 118 by performing speech recognition on the signal indicative of the audible command. According to some implementations, the usage selector 826 causes the screen to display the cartridge data 116 and/or any data (e.g., the usage data 118) associated with usage of the cartridge 102 and/or the AED 104, and/or enables the user to confirm, modify, and/or control the cartridge data 116.

According to various examples, the processor(s) 812 are operably connected to one or more position sensors 828. The position sensor(s) 828 are configured to identify a position, movement, or orientation of the external defibrillator 800. For example, the position sensor(s) 828 include a location service circuit (e.g., a Global Navigation Satellite System (GNSS) circuit, such as a Global Positioning System (GPS) circuit) configured to identify the position of the external defibrillator 800, an accelerometer configured to identify an acceleration of the external defibrillator 800, or a gyroscope configured to identify the orientation of the external defibrillator 800.

The memory 814 further includes a settings selector 830, in various examples. When executed by the processor(s) 812, the settings selector 830 causes the processor(s) 812 to identify and/or manage any settings data (e.g., the settings data as discussed above with reference to FIG. 1).

The settings selector 830, in some implementations, causes the screen to output a settings menu. For instance, the settings menu enable the user to view and/or modify any settings data.

The processor(s) 812 is operably connected to a charging circuit 832 and a discharge circuit 834. In various implementations, the charging circuit 832 includes a power source 836, one or more charging switches 838, and one or more capacitors 840. The power source 836 includes, for instance, a battery. The processor(s) 812 initiates a defibrillation shock by causing the power source 836 to charge at least one capacitor among the capacitor(s) 840. For example, the processor(s) 812 activates at least one of the charging switch(es) 838 in the charging circuit 832 to complete a first circuit connecting the power source 836 and the capacitor to be charged. Then, the processor(s) 812 causes the discharge circuit 834 to discharge energy stored in the charged capacitor across a pair of defibrillation electrodes 842, which are in contact with the individual 808. For example, the processor(s) 812 deactivates the charging switch(es) 838 completing the first circuit between the capacitor(s) 840 and the power source 836, and activates one or more discharge switches 844 completing a second circuit connecting the charged capacitor 840 and at least a portion of the individual 808 disposed between defibrillation electrodes 842.

The energy is discharged from the defibrillation electrodes 842 in the form of a defibrillation shock. For example, the defibrillation electrodes 842 are connected to the skin of the individual 808 and located at positions on different sides of the heart of the individual 808, such that the defibrillation shock is applied across the heart of the individual 808. The defibrillation shock, in various examples, depolarizes a significant number of heart cells in a short amount of time. The defibrillation shock, for example, interrupts the propagation of the shockable rhythm (e.g., VF or pulseless V-Tach) through the heart. In some examples, the defibrillation shock is 200 J or greater with a duration of 0.015 seconds. In some cases, the defibrillation shock has a multiphasic (e.g., biphasic) waveform. The discharge switch(es) 844 are controlled by the processor(s) 812, for example. In various implementations, the defibrillation electrodes 842 are connected to defibrillation connectors 846. The defibrillation connectors 846 are connected to a defibrillation port 848, in implementations.

According to various examples, the defibrillation connectors 846 are removable from the defibrillation port 848. For example, the defibrillation connectors 846 are plugged into the defibrillation port 848. In various examples, individual ones of the defibrillation connectors 846 and/or individual ones of the defibrillation electrodes 842 are utilized to implement, individually or in combination, any corresponding electrode pads of the electrode pads 110, as discussed above with reference to FIG. 1.

In various implementations, the processor(s) 812 is operably connected to one or more transceivers 850 that transmit and/or receive data over one or more communication networks 852. For example, the transceiver(s) 850 includes a network interface card (NIC), a network adapter, a local area network (LAN) adapter, or a physical, virtual, or logical address to connect to the various external devices and/or systems. In various examples, the transceiver(s) 850 includes any sort of wireless transceivers capable of engaging in wireless communication (e.g., radio frequency (RF) communication). For example, the communication network(s) 852 includes one or more wireless networks that include a 3^{rd} Generation Partnership Project (3GPP) network, such as a Long Term Evolution (LTE) radio access network (RAN) (e.g., over one or more LE bands), a New Radio (NR) RAN (e.g., over one or more NR bands), or a combination thereof. In some cases, the transceiver(s) 850 includes other wireless modems, such as a modem for engaging in WI-FI^{®}, WIGIG^{®}, WIMAX^{®}, BLUETOOTH^{®}, or infrared communication over the communication network(s) 852.

The defibrillator 800 is configured to transmit and/or receive data (e.g., ECG data, notation data, impedance data, data indicative of one or more detected heart rhythms of the individual 808, data indicative of one or more defibrillation shocks administered to the individual 808, etc.) with one or more external devices 854 via the communication network(s) 852. The external devices 854 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 852. In some examples, the external device(s) 854 is located remotely from the defibrillator 800, such as at a remote clinical environment (e.g., a hospital). According to various implementations, the processor(s) 812 causes the transceiver(s) 850 to transmit data to the external device(s) 854. In some cases, the transceiver(s) 850 receives data from the external device(s) 854 and the transceiver(s) 850 provide the received data to the processor(s) 812 for further analysis.

In various implementations, the external defibrillator 800 also includes one or more housings, including a housing of the cartridge 102 and a housing (e.g., a housing 856) of the AED 104. For example, individual ones of the housing(s) at least partially enclose any combination(s) of other elements of the external defibrillator 800. For example, the housing 856 encloses the detection circuit 810, the processor(s) 812, the memory 814, the charging circuit 832, the transceiver(s) 850, or any combination thereof. In some cases, the input device(s) 818 and output device(s) 820 extend from an interior space at least partially surrounded by the housing 856 through a wall of the housing 856. In various examples, the housing 856 acts as a barrier to moisture, electrical interference, and/or dust, thereby protecting various components in the external defibrillator 800 from damage.

In some implementations, the external defibrillator 800 is an automated external defibrillator (AED) operated by an untrained user (e.g., a bystander, layperson, etc.) and can be operated in an automatic mode. In automatic mode, the processor(s) 812 automatically identifies a rhythm in the ECG signal, makes a decision whether to administer a defibrillation shock, charges the capacitor(s) 840, discharges the capacitor(s) 840, or any combination thereof. In some cases, the processor(s) 812 controls the output device(s) 820 to output (e.g., display) a simplified user interface to the untrained user. For example, the processor(s) 812 refrains from causing the output device(s) 820 to display a waveform of the ECG signal and/or the impedance signal to the untrained user, in order to simplify operation of the external defibrillator 800.

In some examples, the external defibrillator 800 is a monitor-defibrillator utilized by a trained user (e.g., a clinician, an emergency responder, etc.) and can be operated in a manual mode or the automatic mode. When the external defibrillator 800 operates in manual mode, the processor(s) 812 cause the output device(s) 820 to display a variety of information that may be relevant to the trained user, such as waveforms indicating the ECG data and/or impedance data, notifications about detected heart rhythms, and the like.

### CONCLUSION

The features disclosed in the foregoing description or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification are to be understood as being modified in all instances by the term "about." Accordingly, unless indicated to the contrary, the numerical parameters set forth in the specification are approximations that may vary depending upon the desired properties sought to be obtained by the present disclosure. At the very least each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Moreover, any combination of the above-described elements in all possible variations thereof is encompassed by implementations of the disclosure unless otherwise indicated herein or otherwise clearly contradicted by context.

## Claims

1. A medical device, comprising:
a memory (114);
a cavity configured to receive a cartridge (102, 202, 302) that comprises electrode pads (110), a battery (108), and a storage device;
a conductor configured to route a signal that identifies the cartridge is removably coupled to the cavity;
a processor (812) configured to analyze (604) a signature communicated by the signal that matches a value stored in the memory, the signature being utilized to identify that the cartridge is unused; and
a display (500) configured to, in response to analyzing the signature, switch (606) between illuminating any portion of the display to illuminating an indicator that symbolizes shocking a patient (106).

2. The medical device of claim 1, wherein the display is further configured to illuminate (706) a use indicator (502) with a steady light representing that another cartridge being removably coupled to the cavity is unused, and/or to illuminate a use indicator with a blinking light representing that the cartridge is used.

3. The medical device of claim 1 or 2, wherein the storage device comprises a memory card, read only memory (ROM), flash memory, a micro secure digital (SD) card, a SD card, a compact flash card, programmable read-only memory (PROM), electrically erasable, programmable, read-only memory (EEPROM), a smart card, a subscriber identity module (SIM) card, and/or a radio frequency identification (RFID) chip.

4. The medical device of claim 1, 2 or 3, wherein the processor is further configured to:
receive (602) a communication from the disposable cartridge;
in response to receiving the communication, analyze (604) a flag with a default value representing the disposable cartridge being unused; and
in response to analyzing the flag with the default value, control (606) a display to switch to illuminating an indicator that symbolizes shocking the patient.

5. The medical device of any of claims 1-4, wherein the processor is further configured to:
Receive (704), from the cartridge, an identification signal indicating a serial number of the cartridge.

6. The medical device of any of claims 1-5, wherein a printed circuit board (PCB) is coupled between a first surface of the cartridge and a second surface of the cartridge, and
an adhesive connects the first surface with the second surface by a seal.

7. The medical device of any of claims 1-6, further comprising:
a transceiver (850),
wherein the processor is further configured to:
analyze an identifier received via a communication from the cartridge, the identifier being stored in another storage device of the cartridge and representing an age of the electrode pads or the battery; and
in response to recognizing that the age is greater than a threshold, control the transceiver to transmit an alert.

8. The medical device of any of claims 1-7, wherein the processor is configured to:
receive an identifier representing an age of the electrode pads or the battery of the cartridge; and
in response to recognizing that the age is greater than a threshold, control presenting by the display of an alert.

9. The medical device of any of claims 1-8, further comprising:
a sensor configured to sense that a potential at a port of the cavity is modified in response to coupling the port with a pin of a connector of the cartridge,
wherein analyzing the signature comprises analyzing the signature in response to the sensing that the potential is modified.

10. A system, comprising:
a disposable cartridge (202) comprising:
a battery (108);
electrode pads (110) configured to be disposed on a chest of a patient (106); and
a storage device storing an indication of a status of the disposable cartridge; a sensor configured to detect the status of the disposable cartridge, the status comprising a charge level of the battery or whether a charge has been received by the electrode pads; and
a medical device according to any of claims 1-9, the medical device comprising an automated external defibrillator (AED) (104, 204, 306, 400, 800) configured to be powered by the disposable cartridge, the AED comprising:
a measurement circuit (810) configured to detect an electrocardiogram (ECG) of the patient; and
a treatment circuit (834) configured to output an electrical shock;
wherein the processor is further configured to:
determine, by analyzing the status of the disposable cartridge, that the disposable cartridge is ready to use; and
in response to determining that the disposable cartridge is ready to use:
cause the measurement circuit to be electrically coupled to the electrode pads in the disposable cartridge;
cause the treatment circuit to be electrically coupled to the electrode pads in the disposable cartridge; and
cause the treatment circuit to be electrically coupled to the battery in the disposable cartridge.

11. A method (600), comprising:
receiving (602) a signal that identifies a cartridge (102, 202, 302) removably coupled to a cavity of a medical device, the cartridge comprising electrode pads (110), a battery (108), and a storage device;
analyzing (604) a signature communicated by the signal that matches a value stored in a memory (114) of the medical device, the signature being utilized to identify that the cartridge is unused; and
in response to analyzing the signature, controlling (606) a display of the medical device to switch between illuminating any portion of the display to illuminating an indicator that symbolizes shocking a patient (106).

12. The method of claim 11, further comprising:
controlling the display to illuminate (706) a use indicator (502) with a steady light representing that the cartridge is unused, and/or
controlling the display to illuminate a use indicator with a blinking light representing that another cartridge being removably coupled to the cavity is used.

13. The method of claim 11 or 12, further comprising:
analyzing an identifier received via the signal that represents an age of the electrode pads or a battery; and
in response to recognizing that the age is greater than a threshold, controlling a transceiver to transmit an alert.

14. The method of any of claims 11-13, wherein controlling the display further comprises:
setting a flag identifying a first electrode pad that is positioned on a chest located on the patient and a second electrode pad that is positioned on a midsection located on the patient;
identifying an electrocardiogram (ECG) characteristic in response to the setting of the flag;
analyzing an amplitude represented by the ECG characteristic;
confirming the amplitude is greater than a threshold; and
controlling the display to illuminate the indicator in response to confirming the amplitude is greater than the threshold.

15. The method of any of claims 11-14, wherein controlling the display comprises setting the display to indicate that no mode is set;
receiving a selection via input to the display that energizes utilizing the medical device for the patient that is a child;
controlling the display to indicate placement of the electrode pads on the child;
identifying a potential being changed that represents the electrode pads being positioned on the child; and
controlling the display to switch to illuminating the indicator that symbolizes shocking the patient in response to identifying the potential being changed that represents the electrode pads being positioned on the child.

## Patentansprüche

1. Medizinische Vorrichtung, die Folgendes umfasst:
einen Speicher (114);
einen Hohlraum, der konfiguriert ist, eine Kartusche (102, 202, 302) aufzunehmen, die Elektrodenpads (110), eine Batterie (108) und eine Speichervorrichtung umfasst;
einen Leiter, der konfiguriert ist, ein Signal zu leiten, das die Kartusche identifiziert, die abnehmbar mit dem Hohlraum gekoppelt ist;
einen Prozessor (812), der konfiguriert ist, eine Signatur zu analysieren (604), die durch das Signal übertragen wird, die mit einem in dem Speicher gespeicherten Wert übereinstimmt, wobei die Signatur verwendet wird, um zu identifizieren, dass die Kartusche unbenutzt ist; und
eine Anzeige (500), die konfiguriert ist, in Reaktion auf das Analysieren der Signatur zwischen dem Beleuchten eines beliebigen Teils der Anzeige zum Beleuchten eines Indikators, der ein Schocken eines Patienten (106) symbolisiert, umzuschalten (606).

2. Medizinische Vorrichtung nach Anspruch 1, wobei die Anzeige ferner konfiguriert ist, einen Verwendungsindikator (502) mit einem Dauerlicht zu beleuchten (706), das repräsentiert, dass eine weitere Kartusche, die abnehmbar mit dem Hohlraum gekoppelt ist, unbenutzt ist, und/oder einen Verwendungsindikator mit einem Blinklicht zu beleuchten, das repräsentiert, dass die Kartusche verwendet wird.

3. Medizinische Vorrichtung nach Anspruch 1 oder 2, wobei die Speichervorrichtung eine Speicherkarte, Festwertspeicher (ROM), Flash-Speicher, eine sichere digitale Mikrokarte (microSD-Karte), eine SD-Karte, eine CompactFlash-Karte, programmierbaren Festwertspeicher (PROM), elektrisch löschbaren programmierbaren Festwertspeicher (EEPROM), eine Chipkarte, eine Teilnehmeridentitätsmodul-Karte (SIM-Karte) und/oder einen Hochfrequenzidentifikations-Chip (RFID-Chip) umfasst.

4. Medizinische Vorrichtung nach Anspruch 1, 2 oder 3, wobei der Prozessor ferner konfiguriert ist:
eine Übertagung von der Einwegkartusche zu empfangen (602);
in Reaktion auf das Empfangen der Übertragung einen Merker mit einem Standardwert, der repräsentiert, dass die Einwegkartusche unbenutzt ist, zu analysieren (604); und
in Reaktion auf das Analysieren des Merkers mit dem Standardwert eine Anzeige zu steuern (606), um zum Beleuchten eines Indikators umzuschalten, der ein Schocken des Patienten symbolisiert.

5. Medizinische Vorrichtung nach einem der Ansprüche 1-4, wobei der Prozessor ferner konfiguriert ist:
ein Identifikationssignal, das eine Seriennummer der Kartusche angibt, von der Kartusche zu empfangen (704).

6. Medizinische Vorrichtung nach einem der Ansprüche 1-5, wobei eine Leiterplatte (PCB) zwischen eine erste Oberfläche der Kartusche und eine zweite Oberfläche der Kartusche gekoppelt ist, und
ein Klebstoff die erste Oberfläche durch eine Dichtung mit der zweiten Oberfläche verbindet.

7. Medizinische Vorrichtung nach einem der Ansprüche 1-6, die ferner Folgendes umfasst:
einen Sendeempfänger (850),
wobei der Prozessor ferner konfiguriert ist:
eine Kennung zu analysieren, die über eine Übertragung von der Kartusche empfangen wird, wobei die Kennung in einer weiteren Speichervorrichtung der Kartusche gespeichert ist und ein Alter der Elektrodenpads oder der Batterie repräsentiert; und
in Reaktion auf das Erkennen, dass das Alter größer als einen Schwellenwert ist, den Sendeempfänger zu steuern, um eine Warnung zu übertragen.

8. Medizinische Vorrichtung nach einem der Ansprüche 1-7, wobei der Prozessor konfiguriert ist:
eine Kennung zu empfangen, die ein Alter der Elektrodenpads oder der Batterie der Kartusche repräsentiert; und
in Reaktion auf das Erkennen, dass das Alter größer als ein Schwellenwert ist, das Darstellen einer Warnung durch die Anzeige zu steuern.

9. Medizinische Vorrichtung nach einem der Ansprüche 1-8, die ferner Folgendes umfasst:
einen Sensor, der konfiguriert ist, zu erfassen, dass ein Potential an einem Anschluss des Hohlraums in Reaktion auf das Koppeln des Anschlusses mit einem Stift eines Verbinders der Kartusche modifiziert wird,
wobei das Analysieren der Signatur das Analysieren der Signatur in Reaktion auf das Erfassen, dass das Potenzial modifiziert worden ist, umfasst.

10. System, das Folgendes umfasst:
eine Einwegkartusche (202), die Folgendes umfasst:
eine Batterie (108);
Elektrodenpads (110), die konfiguriert sind, auf einer Brust eines Patienten (106) angeordnet zu werden; und
eine Speichervorrichtung, die eine Angabe eines Status der Einwegkartusche speichert; einen Sensor, der konfiguriert ist, den Status der Einwegkartusche zu detektieren, wobei der Status einen Ladepegel der Batterie umfasst oder umfasst, ob eine Ladung durch die Elektrodenpads empfangen worden ist; und
ein medizinische Vorrichtung nach einem der Ansprüche 1-9, wobei die medizinische Vorrichtung einen automatisierten externen Defibrillator (AED) (104, 204, 306, 400, 800) umfasst, der konfiguriert ist, durch die Einwegkartusche mit Leistung versorgt zu werden, wobei der AED Folgendes umfasst:
eine Messschaltung (810), die konfiguriert ist, ein Elektrokardiogramm (EKG) des Patienten zu detektieren; und
eine Behandlungsschaltung (834), die konfiguriert ist, einen elektrischen Schock auszugeben;
wobei der Prozessor ferner konfiguriert ist:
durch das Analysieren des Status der Einwegkartusche zu bestimmen, dass die Einwegkartusche einsatzbereit ist; und
in Reaktion auf die Bestimmung, dass die Einwegkartusche einsatzbereit ist:
zu verursachen, dass die Messschaltung mit den Elektrodenpads an der Einwegkartusche elektrisch gekoppelt wird;
zu verursachen, dass die Behandlungsschaltung mit den Elektrodenpads an der Einwegkartusche elektrisch gekoppelt wird; und
zu verursachen, dass die Behandlungsschaltung mit der Batterie in der Einwegkartusche elektrisch gekoppelt wird.

11. Verfahren (600), das Folgendes umfasst:
Empfangen (602) eines Signals, das eine Kartusche (102, 202, 302) identifiziert, die abnehmbar mit einem Hohlraum einer medizinischen Vorrichtung gekoppelt ist, wobei die Kartusche die Elektrodenpads (110), eine Batterie (108) und eine Speichervorrichtung umfasst;
Analysieren (604) einer Signatur, die durch das Signal übertragen wird, die mit einem Wert übereinstimmt, der in einem Speicher (114) der medizinischen Vorrichtung gespeichert ist, wobei die Signatur verwendet wird, um zu identifizieren, dass die Kartusche unbenutzt ist; und
in Reaktion auf das Analysieren der Signatur Steuern (606) einer Anzeige der medizinischen Vorrichtung, um zwischen dem Beleuchten eines beliebigen Teils der Anzeige zum Beleuchten eines Indikators, der ein Schocken eines Patienten (106) symbolisiert, umzuschalten .

12. Verfahren nach Anspruch 11, das ferner Folgendes umfasst:
Steuern der Anzeige, um einen Gebrauchsindikator (502) mit einem Dauerlicht zu beleuchten (706), das repräsentiert, dass die Kartusche unbenutzt ist, und/oder Steuern der Anzeige, um einen Gebrauchsindikator mit einem Blinklicht zu beleuchten, das repräsentiert, dass eine weitere Kartusche, die abnehmbar mit dem Hohlraum gekoppelt ist, verwendet wird.

13. Verfahren nach Anspruch 11 oder 12, das ferner Folgendes umfasst:
Analysieren einer Kennung, die über das Signal empfangen wird, die ein Alter der Elektrodenpads oder einer Batterie repräsentiert; und
in Reaktion auf das Erkennen, dass das Alter größer als ein Schwellenwert ist, Steuern eines Sendeempfängers, um eine Warnung zu übertragen.

14. Verfahren nach einem der Ansprüche 11 bis 13, wobei das Steuern der Anzeige ferner Folgendes umfasst:
Setzen eines Merkers, der ein erstes Elektrodenpad, das auf einer Brust positioniert ist, die sich auf dem Patienten befindet, und ein zweites Elektrodenpad, das auf einem Mittelabschnitt positioniert ist, der sich auf dem Patienten befindet, identifiziert;
Identifizieren einer Elektrokardiogramm-Kennlinie (EKG-Kennlinie) in Reaktion auf das Setzen des Merkers;
Analysieren einer durch die EKG-Kennlinie repräsentierten Amplitude;
Bestätigen, dass die Amplitude größer als ein Schwellenwert ist; und
Steuern der Anzeige, um den Indikator zu beleuchten, in Reaktion auf die Bestätigung, dass die Amplitude größer als der Schwellenwert ist.

15. Verfahren nach einem der Ansprüche 11-14, wobei das Steuern der Anzeige das Einstellen der Anzeige umfasst, um anzugeben, dass keine Betriebsart eingestellt ist; Empfangen einer Auswahl über eine Eingabe in die Anzeige, die die Verwendung der medizinischen Vorrichtung für den Patienten, der ein Kind ist, aktiviert;
Steuern der Anzeige, um eine Plazierung der Elektrodenpads auf dem Kind anzugeben;
Identifizieren eines Potentials, das sich geändert hat, das repräsentiert, dass die Elektrodenpads auf dem Kind positioniert sind; und
Steuern der Anzeige, um zum Beleuchten des Indikators umzuschalten, der das Schocken des Patienten symbolisiert, in Reaktion auf das Identifizieren, dass sich das Potential geändert hat, das repräsentiert, dass die Elektrodenpads auf dem Kind positioniert sind.

## Revendications

1. Dispositif médical, comprenant :
une mémoire (114) ;
une cavité configurée pour recevoir une cartouche (102, 202, 302) qui comprend des pastilles d'électrodes (110), une batterie (108), et un dispositif de stockage ;
un conducteur configuré pour acheminer un signal qui identifie que la cartouche est accouplée de manière amovible à la cavité ;
un processeur (812) configuré pour analyser (604) une signature communiquée par le signal qui correspond à une valeur stockée dans la mémoire, la signature étant utilisée pour identifier que la cartouche est inutilisée ; et
un afficheur (500) configuré pour, en réponse à l'analyse de la signature, basculer (606) entre l'éclairage d'une partie quelconque de l'afficheur et l'éclairage d'un témoin qui symbolise le choc d'un patient (106).

2. Dispositif médical selon la revendication 1, dans lequel l'afficheur est en outre configuré pour éclairer (706) un témoin d'utilisation (502) avec une lumière fixe représentant le fait qu'une autre cartouche accouplée de manière amovible à la cavité est inutilisée, et/ou pour éclairer un témoin d'utilisation avec une lumière clignotante représentant le fait que la cartouche est utilisée.

3. Dispositif médical selon la revendication 1 ou 2, dans lequel le dispositif de stockage comprend une carte mémoire, une mémoire morte (ROM), une mémoire flash, une carte mémoire Secure Digital (SD), une carte SD, une carte flash compacte, une mémoire morte programmable (PROM), une mémoire morte programmable effaçable électriquement (EEPROM), une carte à puce, une carte de module d'identification d'abonné (SIM) et/ou une puce d'identification par radiofréquence (RFID).

4. Dispositif médical selon la revendication 1, 2 ou 3, dans lequel le processeur est en outre configuré pour :
recevoir (602) une communication provenant de la cartouche jetable ;
en réponse à la réception de la communication, analyser (604) un indicateur ayant une valeur par défaut représentant la cartouche jetable inutilisée ; et
en réponse à l'analyse de l'indicateur ayant la valeur par défaut, commander (606) un afficheur pour commuter sur l'éclairage d'un témoin qui symbolise le choc du patient.

5. Dispositif médical selon l'une quelconque des revendications 1 à 4, dans lequel le processeur est en outre configuré pour :
recevoir (704), de la cartouche, un signal d'identification indiquant un numéro de série de la cartouche.

6. Dispositif médical selon l'une quelconque des revendications 1 à 5, dans lequel une carte de circuit imprimé (PCB) est couplée entre une première surface de la cartouche et une seconde surface de la cartouche, et un adhésif relie la première surface à la seconde surface par un joint d'étanchéité.

7. Dispositif médical selon l'une quelconque des revendications 1 à 6, comprenant en outre :
un émetteur-récepteur (850),
dans lequel le processeur est en outre conçu pour :
analyser un identifiant reçu par le biais d'une communication provenant de la cartouche, l'identifiant étant stocké dans un autre dispositif de stockage de la cartouche et représentant un âge des pastilles d'électrodes ou de la batterie ; et
en réponse à la reconnaissance que l'âge est supérieur à un seuil, commander l'émetteur-récepteur pour émettre une alerte.

8. Dispositif médical selon l'une quelconque des revendications 1 à 7, dans lequel le processeur est configuré pour :
recevoir un identifiant représentant un âge des pastilles d'électrode ou de la batterie de la cartouche ; et
en réponse à la reconnaissance que l'âge est supérieur à un seuil, commander la présentation d'une alerte par l'afficheur.

9. Dispositif médical selon l'une quelconque des revendications 1 à 8, comprenant en outre :
un capteur configuré pour détecter qu'un potentiel au niveau d'un orifice de la cavité est modifié en réponse au couplage du port avec une broche d'un connecteur de la cartouche,
dans lequel l'analyse de la signature comprend l'analyse de la signature en réponse à la détection que le potentiel est modifié.

10. Système comprenant :
une cartouche jetable (202) comprenant :
une batterie (108) ;
des pastilles d'électrodes (110) configurées pour être disposées sur la poitrine d'un patient (106) ; et
un dispositif de stockage stockant une indication d'un état de la cartouche jetable ; un capteur configuré pour détecter l'état de la cartouche jetable, l'état comprenant un niveau de charge de la batterie ou si une charge a été reçue ou non par les pastilles d'électrodes ; et
un dispositif médical selon l'une quelconque des revendications 1 à 9, le dispositif médical comprenant un défibrillateur automatisé externe (DAE) (104, 204, 306, 400, 800) configuré pour être alimenté par la cartouche jetable, le DAE comprenant :
un circuit de mesure (810) configuré pour détecter un électrocardiogramme (ECG) du patient ; et
un circuit de traitement (834) configuré pour sortir un choc électrique ;
dans lequel le processeur est en outre conçu pour :
déterminer, en analysant l'état de la cartouche jetable, que la cartouche jetable est prête à l'emploi ; et
en réponse à la détermination que la cartouche jetable est prête à l'emploi :
faire en sorte que le circuit de mesure soit couplé électriquement aux pastilles d'électrodes dans la cartouche jetable ;
faire en sorte que le circuit de traitement soit couplé électriquement aux pastilles d'électrodes dans la cartouche jetable ; et
faire en sorte que le circuit de traitement soit couplé électriquement à la batterie dans la cartouche jetable.

11. Procédé (600), comprenant :
la réception (602) d'un signal qui identifie une cartouche (102, 202, 302) accouplée de manière amovible à une cavité d'un dispositif médical, la cartouche comprenant des pastilles d'électrodes (110), une batterie (108) et un dispositif de stockage ;
l'analyse (604) d'une signature communiquée par le signal qui correspond à une valeur stockée dans une mémoire (114) du dispositif médical, la signature étant utilisée pour identifier que la cartouche est inutilisée ; et
en réponse à l'analyse de la signature, la commande (606) d'un afficheur du dispositif médical pour commuter entre l'éclairage d'une partie quelconque de l'afficheur et l'éclairage d'un témoin qui symbolise le choc d'un patient (106).

12. Procédé selon la revendication 11, comprenant en outre :
la commande de l'afficheur pour éclairer (706) un témoin d'utilisation (502) avec une lumière fixe représentant le fait que la cartouche n'est pas utilisée, et/ou la commande de l'afficheur pour éclairer un témoin d'utilisation avec une lumière clignotante représentant le fait qu'une autre cartouche accouplée de manière amovible à la cavité est utilisée.

13. Procédé selon la revendication 11 ou 12, comprenant en outre :
l'analyse d'un identifiant reçu par le biais du signal qui représente un âge des pastilles d'électrodes ou d'une batterie ; et
en réponse à la reconnaissance que l'âge est supérieur à un seuil, la commande d'un émetteur-récepteur pour émettre une alerte.

14. Procédé selon l'une quelconque des revendications 11 à 13, dans lequel la commande de l'afficheur comprend en outre :
le réglage d'un indicateur identifiant une première pastille d'électrode qui est positionnée sur la poitrine du patient et une seconde pastille d'électrode qui est positionnée sur le tronc du patient ;
l'identification d'une caractéristique d'électrocardiogramme (ECG) en réponse au réglage de l'indicateur ;
l'analyse d'une amplitude représentée par la caractéristique d'ECG ;
la confirmation que l'amplitude est supérieure à un seuil ; et
la commande de l'afficheur pour allumer le témoin en réponse à la confirmation que l'amplitude est supérieure au seuil.

15. Procédé selon l'une quelconque des revendications 11 à 14, dans lequel la commande de l'afficheur comprend le réglage de l'afficheur pour indiquer qu'aucun mode n'est réglé ;
la réception d'une sélection par le biais d'une entrée dans l'afficheur qui active l'utilisation du dispositif médical pour le patient qui est un enfant ;
la commande de l'afficheur pour indiquer le placement des pastilles d'électrodes sur l'enfant ;
l'identification d'un potentiel étant modifié qui représente le fait que les pastilles d'électrodes sont positionnées sur l'enfant ; et
la commande de l'afficheur pour basculer vers l'éclairage du témoin qui symbolise le choc du patient en réponse à l'identification que le potentiel qui représente les pastilles d'électrodes placées sur l'enfant est modifié.
